# EUROPÄISCHE PATENTSCHRIFT

(11) **EP 1 778 680 B1**
(45) Veröffentlichungstag und Bekanntmachung des Hinweises auf die Patenterteilung: **08.10.2008**
(21) Anmeldenummer: 05776804.6
(22) Anmeldetag: 09.08.2005
(51) Int. Cl.: C07D 409/04, C07D 471/04, A61K 31/437, A61K 31/4355

(54) **SPIROCYCLISCHE CYCLOHEXAN-DERIVATE**
SPIROCYCLIC CYCLOHEXANE DERIVATIVES
DERIVES DE CYCLOHEXANE SPIROCYCLIQUES

(30) Priorität: 13.08.2004 DE 102004039382
(43) Veröffentlichungstag der Anmeldung: 02.05.2007
(73) Patentinhaber: Grünenthal GmbH, 52078 Aachen (DE)
(72) Erfinder: SUNDERMANN, Bernd, 52074 Aachen (DE); HINZE, Claudia, 52066 Aachen (DE); SCHICK, Hans, 13086 Berlin- Weissensee (DE); SONNENSCHEIN, Helmut, 10245 Berlin (DE)
(74) Vertreter: Bülle, Jan
(86) Internationale Anmeldenummer: PCT/EP2005/008625
(87) Internationale Veröffentlichungsnummer: WO 2006/018184

(56) Entgegenhaltungen:
- EP-A- 1 142 587
- WO-A-20/04043967
- WO-A-20/05033112

## Beschreibung

Die vorliegende Erfindung betrifft spirocyclische Cyclohexan-Derivate, Verfahren zu deren Herstellung, Arzneimittel enthaltend diese Verbindungen und die Verwendung von spirocyclischen Cyclohexan-Derivaten zur Herstellung von Arzneimitteln.

Die Behandlung chronischer und nichtchronischer Schmerzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände,
wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation und Toleranzentwicklung limitiert. Außerdem sind sie bei neuropathischen oder inzidentiellen Schmerzen, unter denen insbesondere Tumorpatienten leiden, weniger wirksam.

Aus der Literatur sind bereits Aminomethylcyclohexanderivate zur Behandlung von Schmerz bekannt: WO 0230870 beschreibt substituierte C-Cyclohexylmethylamin-Derivate, die sich zur Behandlung von Schmerz eignen. Diese Verbindungen sind ebenfalls in 4-Position des Cyclohexanringes substituiert, sind jedoch immer über eine Einfach- oder eine Doppelbindung mit dem entsprechenden Rest verknüpft und bilden keinen Spirozyklus mit dem Cyclohexanring.

Aufgabe der vorliegenden Erfindung war es, Arzneimittel zur Verfügung zu stellen, die auf das Opioid-Rezeptor-System wirken und damit für Arzneimittel insbesondere zur Behandlung der verschiedenen mit diesem System nach dem Stand der Technik in Verbindung stehenden Krankheiten bzw. zum Einsatz in den dort genannten Indikationen geeignet sind.

Gegenstand der Erfindung sind daher spirocyclische Cyclohexan-Derivate der allgemeinen Formel I, 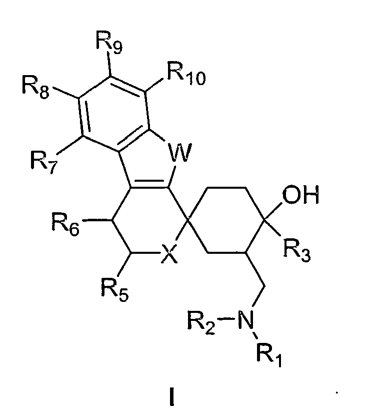 , worin
R¹ und R², unabhängig voneinander für H; CHO; C₁₋₅-Alkyl jeweils gesättigt oder ungesättigt, verzweigt oder unverzweigt, einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, stehen;
oder die Reste R¹ und R² zusammen für CH₂CH₂OCH₂CH₂, CH₂CH₂NR¹¹ CH₂CH₂ oder (CH₂)₃₋₆ stehen,
   wobei R¹¹ H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, bedeutet;
R³ für Phenyl, Phenethyl, Thiophenyl, Pyridyl oder Benzyl, jeweils unsubstituiert oder einfach oder mehrfach substituiert, steht;
W für NR⁴, O oder S steht
   und
   R⁴ für H; C₁₋₅-Alkyl, gesättigt oder ungesättigt, verzweigt oder unverzweigt, unsubstituiert oder einfach oder mehrfach substituiert; Aryl, oder Heteroaryl, jeweils substituiert oder unsubstituiert; über eine C₁₋₃-Alkyl-Gruppe gebundenes Aryl, Heteroaryl oder Cycloalkyl, jeweils einfach oder mehrfach substituiert oder unsubstituiert; COR¹² ; SO₂R¹² steht,
      wobei R¹² H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert; OR¹³; NR¹⁴R¹⁵ bedeutet;
R⁵ für =O; H; COOR¹³, CONR¹³, OR¹³; C₁₋₅-Alkyl, gesättigt oder ungesättigt, verzweigt oder unverzweigt, unsubstituiert oder einfach oder mehrfach substituiert; C₃₋₈-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, steht;
R⁶ für H; F, Cl, NO₂, CF₃, OR¹³, SR¹³, SO₂R¹³ SO₂OR¹³, CN, COOR¹³ NR¹⁴R¹⁵; C₁₋₅-Alkyl, gesättigt oder ungesättigt, verzweigt oder unverzweigt, unsubstituiert oder einfach oder mehrfach substituiert; C₃₋₈-Cycloal, gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, steht;
oder R⁵ und R⁶ gemeinsam (CH₂)ₙ mit n = 2, 3, 4, 5 oder 6 bedeuten, wobei einzelnen Wasserstoffatome auch durch F, Cl, Br, I, NO₂, CF₃, OR¹³, CN oder C₁₋₅-Alkyl ersetzt sein können;
R⁷, R⁸, R⁹ und R¹⁰ unabhängig voneinander für H, F, Cl, Br, I, NO₂, CF₃, OR¹³, SR¹³, SO₂R¹³, SO₂OR¹³, CN, COOR¹³, NR¹⁴R¹⁵; C₁₋₅-Alkyl, C₃₋₈-Cycloalkyl, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, stehen;
   wobei R¹³ H; C₁₋₅-Alkyl jeweils gesättigt oder ungesättigt, verzweigt oder unverzweigt, unsubstituiert oder einfach oder mehrfach substituiert; C₃₋₈-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, bedeutet;
   R¹⁴ und R¹⁵ unabhängig voneinander H; C₁₋₅-Alkyl , jeweils gesättigt oder ungesättigt, verzweigt oder unverzweigt, unsubstituiert oder einfach oder mehrfach substituiert; oder C₃₋₈ -Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, bedeuten;
   oder R¹⁴ und R¹⁵ zusammen CH₂CH₂OCH₂CH₂, CH₂CH₂NR¹⁶CH₂CH₂ oder (CH₂)₃₋₆ bilden,
   wobei R¹⁶ H; C₁₋₅-Alkyl gesättigt oder ungesättigt, verzweigt oder unverzweigt, unsubstituiert oder einfach oder mehrfach substituiert, bedeutet;
X für O, S, SO, SO₂ oder NR¹⁷ steht;
   R¹⁷ für H; C₁₋₅-Alkyl, gesättigt oder ungesättigt, verzweigt oder unverzweigt; COR¹² oder SO₂R¹² steht,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Bei der Zusammenfassung verschiedener Reste, beispielsweise R⁷, R⁸, R⁹ und R¹⁰ sowie der Zusammenfassung von Resten an deren Substituenten, wie z. B. OR¹³, SR¹³, SO2R¹³ oder COOR¹³, kann ein Substituent, z.B. R¹³, für zwei oder mehrere Reste, beispielsweise R⁷, R⁸, R⁹ und R¹⁰, innerhalb einer Substanz unterschiedliche Bedeutungen annehmen.

Die erfindungsgemäßen Verbindungen zeigen gute Bindung an den µ-Rezeptor. Überraschenderweise zeigte es sich, dass die Substanzen auch die Noradrenalin-und Serotonin-Wiederaufnahme hemmen.

Die Ausdrücke "C₁₋₅-Alkyl" und "C₁₋₃-Alkyl" umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1, 2, 3, 4 oder 5 C-Atomen bzw. 1, 2 oder 3 G-Atomen, d.h. C₁₋₅-Alkanyle, C₂₋₅-Alkenyls und G₂₋₅-Alkinyle bzw. C₁₋₃-Alkanyle, C₂₋₃-Alkenyle und C₂₋₃-Alkinyle. Dabei weisen Alkenyle mindestens eine C-C-Doppelbindung und Alkenyle mindestens eine C-C-Dreifachbindung auf. Vorteilhaft ist Alkyl aus der Gruppe ausgewählt, die Methyl, Ethyl, n-Propyyl, 2-Propyl, n-Butyl, iso-Butyl, sec.-Butyl, tert.-Butyl, n-Pentyl, iso-Pentyl, neo-Pentyl, n-Hexyl, 2-hexyl; Ethylenyl (Vinyl), Ethinyl, Propenyl (-CH₂CH=CH₂, -CH=CH-CH₃, -C(=CH₂)-CH₃), Propinyl (-CH-C≡CH, - C≡C-CH₃), 1,1-Dimethylethyl, 1,1-Dimethylpropyl, Butenyl- Butinyl, Pentenyl und Pentinyl, umfasst.

Im Zusammenhang mit "Alkyl" versteht man unter dem Begriff "substituiert" im Sinne dieser Erfindung die Substitution eines oder mehrerer Wasserstoffreste durch F, Cl, Br, I, -CN, NH₂, NH-Alkyl, NH-Aryl, NH-Heteroaryl, NH-Cycloalkyl, NH-Alkyl-Aryl, NH-Alkyl-Heteroaryl, NH-Alkyl-OH, N(Alkyl)₂, N(Alkyl-Aryl)₂, N(Alkyl-Heteroaryl)₂, N(Cycloalkyl)₂, N(Alkyl-OH)₂, NO₂, SH, S-Alkyl, S-Aryl, S-Heteroaryl, S-Alkyl-Aryl, S-Alkyl-Heteroaryl, S-Cycloalkyl, S-Alkyl-OH, S-Alkyl-SH, OH, O-Alkyl, O-Aryl, O-Heteroaryl, O-Alkyl-Aryl, O-Alkyl-Heteroaryl, O-Cycloalkyl, O-Alkyl-OH, CHO, C(=O)C₁₋₆-Alkyl, C(=S)C₁₋₆-Alkyl, C(=O)Aryl, C(=S)Aryl, C(=O)C₁₋₆-Alkyl-Aryl, C(=O)C₁₋₆-Alkyl-Heteroaryl, C(=S)C₁₋₆-Alkyl-Aryl, C(=S)C₁₋₆-Alkyl-Heteroaryl C(=O)-Heteroaryl, C(=S)-Heteroaryl, C(=O)-Cycloalkyl, C(=S)-Cycloalkyl, CO₂H CO₂-Alkyl CO₂-Alkyl-Aryl, CO₂-Alkyl-Heteroaryl, C(=O)NH₂, C(=O)NH-Alkyl, C(=O)NHAryl, C(=O)NHHeteroaryl, C(=O)NH-Cycloalkyl, C(=O)N(Alkyl)₂, C(=O)N(Alkyl-Aryl)₂, C(=O)N(Alkyl-Heteroaryl)₂, C(=O)N(Cycloalkyl)₂, SO-Alkyl, SO₂-Alkyl, SO₂NH₂, SO₃H PO(O-C₁-₆-Alkyl), Cycloalkyl, Aryl oder Heteroaryl, wobei unter mehrfach substituierten Resten solche Reste zu verstehen sind, die entweder an verschiedenen oder an gleichen Atomen mehrfach, z. B. zwei- oder dreifach, substituiert sind, beispielsweise dreifach am gleichen C-Atom wie im Falle von CF₃ oder -CH₂CF₃ oder an verschiedenen Stellen wie im Falle von -CH(OH)-CH=CH-CHCl₂. Die Mehrfachsubstitution kann mit dem gleichen oder mit verschiedenen Substituenten erfolgen. Ggf. kann ein Substituent auch seinerseits substituiert sein; so umfaßt -OAlkyl u.a. auch -O-CH₂-CH₂-O-CH₂-CH₂-OH.

Der Ausdruck "Cycloalkyl" oder "C₃₋₈-Cycloalkyl" bedeutet für die Zwecke dieser Erfindung cyclische Kohlenwasserstoffe mit 3, 4, 5, 6, 7 oder 8 Kohlenstoffatomen, wobei die Kohlenwasserstoffe gesättigt oder ungesättigt (aber nicht aromatisch), unsubstituiert oder ein- oder mehrfach substituiert sein können. In Bezug auf Cycloalkyl umfasst der Begriff auch gesättigte oder ungesättigte (aber nicht aromatische) Cycloalkyl, in denen ein oder zwei Kohlenstoffatome durch ein Heteroatom S, N oder O ersetzt sind. Vorteilhaft ist C₃₋₈-Cycloalkyl aus der Gruppe ausgewählt, die Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, aber auch Tetrahydropyranyl, Dioxanyl, Dioxolanyl, Morpholinyl, Piperidinyl, Piperazinyl, Pyrazolinonyl und Pyrrolidinyl enthält.

Unter dem Begriff (CH₂)₃₋₆ ist -CH₂-CH₂-CH₂-, -CH₂-CH₂-CH₂-CH₂-, -CH₂-CH₂-CH₂-CH₂-CH₂- und CH₂-CH₂-CH₂-CH₂-CH₂-CH₂- zu verstehen.

Der Ausdruck "Aryl" bedeutet im Sinne dieser Erfindung carbocyclische Ringsysteme mit mindestens einem aromatischen Ring, aber ohne Heteroatome in nur einem der Ringe, u.a. Phenyle, Naphthyle und Phenanthrenyle, Fluoranthenyle, Fluorenyle, Indanyle und Tetralinyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Besonders vorteilhaft sind Phenyl- oder Naphthyl-Reste.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein. Der Heterocyclus kann auch Teil eines bi- oder polycyclischen Systems sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Phthalazinyl, Pyrazolyl, Imidazolyl, Thiazolyl, Oxazolyl, Isoxazolyl, Pyridinyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl enthält, wobei die Bindung an die Verbindungen der allgemeinen Struktur I über jedes beliebige und mögliche Ringglied des Heteroaryl-Restes erfolgen kann.

In Bezug auf "Aryl", "Heteroaryl" sowie "Cycloalkyl," versteht man im Sinne dieser Erfindung unter "ein- oder mehrfach substituiert" die ein- oder mehrfache, z.B. zwei-, drei- vier- oder fünffache, vorzugsweise ein- oder zweifache, Substitution eines oder mehrerer Wasserstoffatome des Ringsystems durch F, Cl, Br, 1, CN, NH₂, NH-Alkyl, NH-Aryl, NH-Heteroaryl, NH-Alkyl-Aryl, NH-Alkyl-Heteroaryl, NH-Cycloalkyl, NH-Alkyl-OH, N(Alkyl)₂, N(Alkyl-Aryl)₂, N(Alkyl-Heteroaryl)₂, N(Cycloalkyl)₂, N(Alkyl-OH)₂, NO₂, SH, S-Alkyl, S-Cycloalkyl, S-Aryl, S-Heteroaryl, S-Alkyl-Aryl, S-Alkyl-Heteroaryl, S-Cycloalkyl, S-Alkyl-OH, S-Alkyl-SH, OH, O-Alkyl, O-Cycloalkyl, O-Aryl, O-Heteroaryl, O-Alkyl-Aryl, O-Alkyl-Heteroaryl, O-Cycloalkyl, O-Alkyl-OH, CHO, C(=O)C₁₋₆-Alkyl, C(=S)C₁₋₆-Alkyl, C(=O)Aryl, C(=S)Aryl, C(=O)-C₁₋₆-Alkyl-Aryl, C(=O)-C₁₋₆-Alkyl-Heteroaryl, C(=S)C₁₋₆-Alkyl-Aryl, C(=S)C₁₋₆-Alkyl-Heteroaryl, C(=O)-Heteroaryl, C(=S)-Heteroaryl, C(=O)-Cycloalkyl, C(=S)-Cycloalkyl, CO₂H, CO₂-Alkyl, CO₂-Alkyl-Aryl, CO₂-Alkyl-Heteroaryl, C(=O)NH₂, C(=O)NH-Alkyl, C(=O)NHAryl, C(=O)NH-Cycloalkyl, C(=O)N(Alkyl)₂, C(=O)N(Alkyl-Aryl)₂, C(=O)N(Alkyl-Heteroaryl)₂, C(=O)N(Cycloalkyl)₂, S(O)-Alkyl, S(O)-Aryl, SO₂-Alkyl, SO₂-Aryl, SO₂NH₂, SO₃H, CF₃, =O, =S; Alkyl, Cycloalkyl, Aryl und/oder Heteroaryl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträglichen Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter dem Begriff des physiologisch verträglichen Salzes mit Anionen oder Säuren versteht man im Sinne dieser Erfindung Salze mindestens einer der erfindungsgemäßen Verbindungen - meist, vorzugsweise am Stickstoff, protoniert - als Kation mit mindestens einem Anion, die physiologisch - insbesondere bei Anwendung im Menschen und/oder Säugetier - verträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3-oder 4-Aminobenzoesäure, 2,4,6-Trimethylbenzoesäure, α-Liponsäure, Acetylglycin, Phosphorsäure, Maleinsäure, Malonsäure, Hippursäure und/oder Asparaginsäure. Besonders bevorzugt ist das Hydrochlorid-Salz, das Citrat und das Sesquicitrat.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Für eine bevorzugte Ausführungsform der erfindungsgemäßen spirocyclischen Cyclohexan-Derivate gilt, dass
R¹ und R² unabhängig voneinander für H; C₁₋₅-Alkyl, gesättigt oder ungesättigt, verzweigt oder unverzweigt, einfach oder mehrfach substituiert oder unsubstituiert, stehen;
oder die Reste R¹ und R² zusammen einen Ring bilden und CH₂CH₂OCH₂CH₂, CH₂CH₂NR¹¹ CH₂CH₂ oder (CH₂)₃₋₆ bedeuten,
wobei R¹¹ H; C₁₋₅-Alkyl, gesättigt oder ungesättigt, verzweigt oder unverzweigt, einfach oder mehrfach substituiert oder unsubstituiert, bedeutet.

Besonders bevorzugt sind spirocyclische Cyclohexan-Derivate, worin R¹ und R² unabhängig voneinander für CH₃ oder H stehen, wobei R¹ und R² nicht gleichzeitig H bedeuten.

Ebenfalls bevorzugt sind spirocyclische Cyclohexan-Derivate, worin R³ Phenyl bedeutet, unsubstituiert oder einfach oder mehrfach substituiert, bevorzugt mit F, Cl, CN, OCH₃, SCH₃, OCH₂CH₃, CH₃, CF₃ oder OH einfach oder mehrfach substituiert, insbesondere Phenyl, 3-Methoxyphenyl, 2-Methylphenyl, 4-Trifluormethylphenyl, 4-Methylthiophenyl, 3-Fluorphenyl und 4-Fluorphenyl.

Weiterhin bevorzugt sind spirocyclische Cyclohexan-Derivate, worin W für NR⁴,O oder S und X für NR¹⁷, O oder S steht.

Besonders bevorzugt sind spirocyclische Cyclohexan-Derivate, worin W für NH, O oder S und X für NH, NC(O)CH₃, O oder S steht

Darüber hinaus sind auch spirocyclische Cyclohexan-Derivate bevorzugt, worin R⁷, R⁸, R⁹ und R¹⁰ unabhängig voneinander für H; C₁₋₅-Alkyl, verzweigt oder unverzweigt, unsubstituiert oder ein- oder mehrfach substituiert; F, Cl, Br, l, CF₃, OH, OCH₃, NH₂, COOH, COOCH₃, NHCH₃ oder N(CH₃)₂ oder NO₂ stehen, insbesondere H, F oder OCH₃.

Außerdem bevorzugt sind spirocyclische Cyclohexan-Derivate, worin R⁵ für H, C₁₋₅-Alkyl, verzweigt oder unverzweigt, unsubstituiert oder einfach oder mehrfach substituiert oder COOR¹³ steht und R⁶ für H oder C₁₋₅Alkyl steht, insbesondere R⁵ und R⁶ H bedeuten.

Besonders bevorzugt sind spirocyclische Cyclohexan-Derivate, worin die Reste R⁵-R¹⁰ H bedeuten.

Ganz besonders bevorzugt sind spirocyclische Cyclohexan-Derivate aus der Gruppe
1,1-(2-(Dimethylaminomethyl)-3-hydroxy-3-(3-methoxyphenyl)pentamethylen)-3,4-dihydro-1H-2,9-diazafluoren-dihydrochlorid
1, 1-(2-(Dimethylaminomethyl)-3-hydroxy-3-(3-methoxyphenyl)pentamethylen)-2-acetyl-3,4-dihydro-1H-2,9-diazafluoren-hydrochlorid
1,1-(2-(Dimethylaminomethyl)-3-hydroxy-3-(3-fluorphenyl)-pentamethylen)-3,4-dihydro-1*H*-2,9-diazafluoren citrat
1,1 -(2-(Dimethylaminomethyl)-3-hydroxy-3-phenyl-pentamethylen)-1,3,4,9-tetrahydro-pyrano[3,4-b]indol-sesquicitrat
1,1-(2-(Dimethylaminomethyl)-3-hyd roxy-3-phenyl-pentamethylen)-1,3,4,9-tetrahydro-pyrano[3,4-b]benzofuran-citrat
1,1-(2-(Dimethylaminomethyl)-3-hydroxy-3-(2-methylphenyl)-pentamethylen)-3,4-dihydro-1*H*-2,9-diazafluoren citrat
1,1-(2-(Dimethylaminomethyl)-3-hydroxy-3-phenyl-pentamethylen)-1,3,4,9-tetrahydro-pyrano[3,4-b]benzothiophen-citrat
1,1-(2-(Dimethylaminomethyl)-3-hydroxy-3-(3-fluorphenyl)-pentamethylen)-1,3,4,9-tetrahydro-pyrano[3,4-b]benzofuran-citrat
1,1-(2-(Dimethylaminomethyl)-3-hydroxy-3-phenyl-pentamethylen)-1,3,4,9-tetrahydro-2-thia-9-aza-fluoren-citrat
1,1-(2-(Dimethylaminomethyl)-3-hydroxy-3-(3-fluorphenyl)-pentamethylen)-1,3,4,9-tetrahydro-pyrano[3,4-b]benzothiophen-citrat
1,1-(2-(Dimethylaminomethyl)-3-hydroxy-3-(2-methylphenyl)-pentamethylen)-1,3,4,9-tetrahydro-pyrano[3,4-b]benzothiophen-citrat
1,1-(2-(Dimethylaminomethyl)-3-hydroxy-3-(2-methylphenyl)-pentamethylen)-1,3,4,9-tetrahydro-pyrano[3,4-b]benzofuran-citrat
1,1-(2-(Dimethylaminomethyl)-3-hydroxy-3-(4-trifluormethylphenyl)-pentamethylen)-1,3,4,9-tetrahydro-pyrano[3,4-b]benzofuran-citrat
1,1-(2-(Dimethylaminomethyl)-3-hydroxy-3-(3-fluorphenyl)-pentamethylen)-1,3,4,9-tetrahydro-2-thia-9-aza-fluoren-citrat
1,1-(2-(Dimethylaminomethyl)-3-hydroxy-3-(4-trifluormethylphenyl)-pentamethylen)-3,4-dihydro-1*H*-2,9-diazafluoren dicitrat
1;1-(2-(Dimethylaminomethyl)-3-hydroxy-3-(3-fluorphenyl)-pentamethylen )-7-fluor-3,4-dihydro-1*H*-2,9-diazafluoren-sesquicitrat
1,1-(2-(Dimethylaminomethyl)-3-hydroxy-3-phenyl-pentamethylen)-3,4-dihydro-6-methoxy-1*H*-2,9-diazafluoren citrat
1,1-(2-(Dimethylaminomethyl)-3-hydroxy-3-(4-methylthiophenyl)-pentamethylen)-1,3,4,9-tetrahydro-6-methoxy-pyrano[3,4-b]indol-citrat
1,1-(2-(Dimethylaminomethyl)-3-hydroxy-3-(2-methylphenyl)-pentamethylen)-1,3,4,9-tetrahydro-6-methoxy-pyrano[3,4-b]indol-citrat
1,1-(2-(Dimethylaminomethyl)-3-hydroxy-3-(4-methylthiophenyl)-pentamethylen)-1,3,4,9-tetrahydro-pyrano[3,4-b]indol-citrat
1,1-(2-(Dimethylaminomethyl)-3-hydroxy-3-(3-fluorphenyl)-pentamethylen)-3,4-dihydro-6-methoxy-1*H*-2,9-diazafluoren citrat
1,1-(2-(Dimethylaminomethyl)-3-hydroxy-3-(4-trifluormethylphenyl)-pentamethylen)-3,4-dihydro-6-methoxy-1*H*-2,9-diazafluoren citrat
1,1-(2-(Dimethylaminomethyl)-3-hydroxy-3-(4-methylthiophenyl)-pentamethylen)-1,3,4,9-tetrahydro-2-thia-9-aza-fluoren-sesquicitrat
1,1-(2-(Dimethylaminomethyl)-3-hydroxy-3-(2-methylphenyl)-pentamethylen)-1,3,4,9-tetrahydro-2-thia-9-aza-fluoren-citrat
1,1-(2-(Dimethylaminomethyl)-3-hydroxy-3-(2-methylphenyl)-pentamethylen)-1,3,4,9-tetrahydro-pyrano[3,4-b]indol-citrat
1,1-(2-(Dimethylaminomethyl)-3-hydroxy-3-(4-methylthiophenyl)-pentamethylen)-3,4-dihydro-1*H*-2,9-diazafluoren citrat
1,1-(2-(Dimethylaminomethyl)-3-hydroxy-3-(3-fluorphenyl)-pentamethylen)-1,3,4,9-tetrahydro-pyrano[3,4-b]indol-hydrochlorid
1,1-(2-(Dimethylaminomethyl)-3-hydroxy-3-phenyl-pentamethylen )-3,4-dihydro-1 H-2,9-diazafluoren citrat
1,1-(2-(Dimethylaminomethyl)-3-hydroxy-3-(4-trifluormethylphenyl)-pentamethylen)-1,3,4,9-tetrahydro-pyrano[3,4-b]benzothiophen-citrat
1,1-(2-(Dimethylaminomethyl)-3-hydroxy-3-(4-methylthiophenyl)-pentamethylen)-1,3,4,9-tetrahydro-pyrano[3,4-b]benzofuran-citrat
1,1-(2-(Dimethylaminomethyl)-3-hydroxy-3-(4-methylthiophenyl)-pentamethylen)-1,3,4,9-tetrahydro-pyrano[3,4-b]benzothiophen-citrat
1,1-(2-(Dimethylaminomethyl)-3-hydroxy-3-(2-methylphenyl)-pentamethylen)-7-fluor-3,4-dihydro-1*H*-2,9-diazafluoren citrat
1,1-(2-(Dimethylaminomethyl)-3-hydroxy-3-(4-trifluormethylphenyl)-pentamethylen)-7-fluor-3,4-dihydro-1*H*-2,9-diazafluoren citrat
1,1-(2-(Dimethylaminomethyl)-3-hydroxy-3-(4-methylthiophenyl)-pentamethylen)-7-fluor-3,4-dihydro-1*H*-2,9-diazafluoren citrat
1,1-(2-(Dimethylaminomethyl)-3-hydroxy-3-phenyl-pentamethylen)-7 -fluor-3,4-dihydro-1*H*-2,9-diazafluoren dicitrat
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Die erfindungsgemäßen Substanzen wirken beispielsweise auf den im Zusammenhang mit verschiedenen Erkrankungen relevanten µ-Opioid-Rezeptor, sodass sie sich als pharmazeutischer Wirkstoff in einem Arzneimittel eignen. Ein weiterer Gegenstand der Erfindung sind daher Arzneimittel enthaltend wenigstens ein erfindungsgemäßes spirocyclisches Cyclohexan-Derivat, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen spirocyclischen Cyclohexan-Derivat gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säften, als halbfeste Arzneiformen in Form von Granulaten, Tabletten, Pellets, Patches, Kapseln, Pflastern/Sprühpflastern oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße spirocyclische Cyclohexan-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen spirocyclischen Cyclohexan-Derivate verzögert freisetzen. Die erfindungsgemäßen spirocyclischen Cyclohexan-Derivate können auch in parenteralen Langzeitdepotformen wie z. B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,01 bis 5 mg/kg wenigstens eines erfindungsgemäßen spirocyclischen Cyclohexan-Derivats appliziert.

Für alle vorstehenden Formen der erfindungsgemäßen Arzneimittel ist es bevorzugt, wenn das Arzneimittel neben wenigstens einem spirocyclischen Cyclohexan-Derivat noch einen weiteren Wirkstoff, insbesondere ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittels liegt ein enthaltenes erfindungsgemäßes spirocyclisches Cyclohexan-Derivat als nicht-äquimolare oder äquimolare Mischung der Diastereomere und/oder Enantiomere vor, besonders bevorzugt als reines Diastereomer und/oder Enantiomer.

Der µ-Opioid-Rezeptor, aber auch die anderen Opioid-Rezeptoren, wurden insbesondere im Schmerzgeschehen identifiziert. Entsprechend können, erfindungsgemäße spirocyclische Cyclohexan-Derivate zur Herstellung eines Arzneimittels zur Behandlung von Schmerz, insbesondere von akutem, viszeralem, neuropathischem oder chronischem Schmerz, verwendet werden.

Ein weiterer Gegenstand der Erfindung ist daher die Verwendung eines erfindungsgemäßen spirocyclischen Cyclohexan-Derivats zur Herstellung eines Arzneimittels zur Behandlung von Schmerz, insbesondere von akutem, viszeralem, neuropathischem oder chronischem Schmerz.

Überraschenderweise hat sich gezeigt, dass sich die erfindungsgemäßen spirocyclischen Cyclohexan-Derivate zur Behandlung von Angstzuständen, Depressionen, Epilepsie, cardiovaskulären Erkrankungen, Harninkontinenz, Diarrhöe, Pruritus, Alkohol- und Drogenmißbrauch, Medikamentenabhängigkeit, Entzündungen, Antriebslosigkeit, Bulimie, Anorexie, Katalepsie, zur Verwendung als Lokalanästhetikum, Antiarrythmikum, Antiemetikum, Nootropikum, und zur Vigilanz-und Libidosteigerung eignen.

Ein weiterer Gegenstand der Erfindung ist daher die Verwendung eines erfindungsgemäßen spirocyclischen Cyclohexan-Derivats zur Herstellung eines Arzneimittels zur Behandlung von Angstzuständen, Depressionen, Epilepsie, cardiovaskulären Erkrankungen, Harninkontinenz, Diarrhöe, Pruritus, Alkohol- und Drogenmißbrauch, Medikamentenabhängigkeit, Entzündungen, Antriebslosigkeit, Bulimie, Anorexie, Katalepsie, zur Verwendung als Lokalanästhetikum, Antiarrythmikum, Antiemetikum, Nootropikum, und zur Vigilanz- und Libidosteigerung.

Dabei kann es in einer der vorstehenden Verwendungen bevorzugt sein, wenn ein verwendetes spirocyclisches Cyclohexan-Derivat als nicht-äquimolare oder äquimolare Mischung der Diastereomere und/oder Enantiomere vorliegt, insbesondere als reines Diasteromer und/oder Enantiomer.

Ein weiterer Gegenstand der Erfindung ist ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wiksamen Dosis eines erfindungsgemäßen spirocyclischen Cyclohexan-Derivats, oder eines erfindungsgemäßen Arzneimittels.

Ein weiterer Gegenstand der Erfindung ist ein Verfahren zur Herstellung der erfindungsgemäßen spirocylischen Cyclohexan-Derivate wie in der folgenden Beschreibung und Beispielen ausgeführt.
R⁰¹ und R⁰² die für erfindungsgemäße Verbindungen gemäß Formel I für R¹ und R² angegebene Bedeutung haben und zusätzlich unabhängig voneinander für eine Schutzgruppe stehen können: 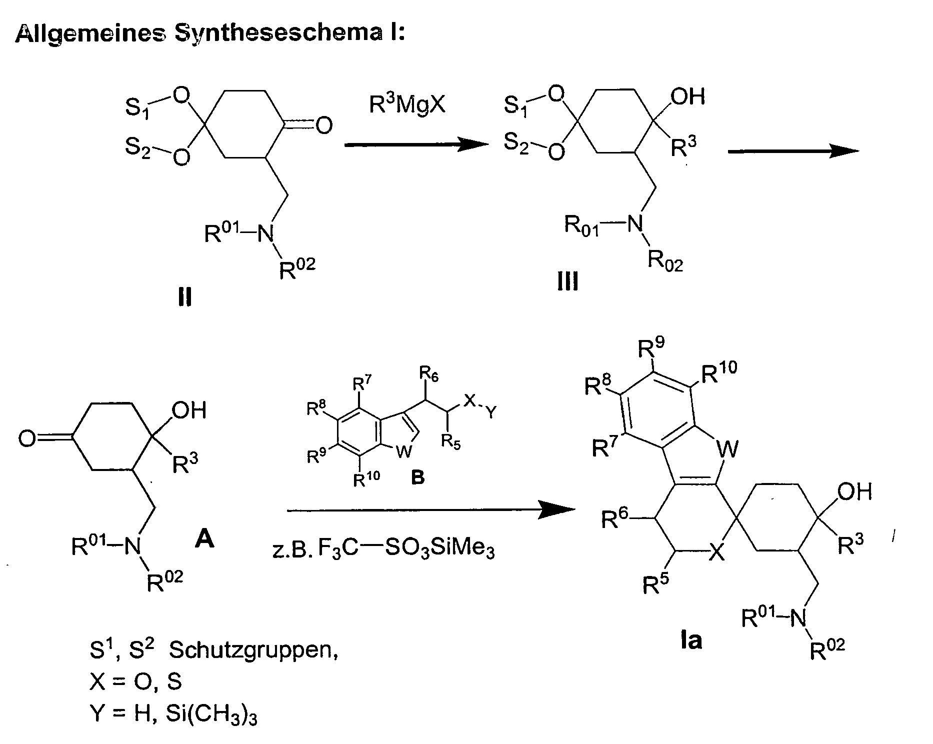 Die Synthese von 7-Dialkylaminomethyl-1,4-dioxa-spiro[4.5]decan-8-on ist in EP0780369 für R¹, R² = Methyl vorbeschrieben.

Eine Mannichbase der allgemeinen Formel II, wobei S1 und S2 für Schutzgruppen, vorzugsweise für eine Ketatschutzgruppe, stehen, wird bei einer Temperatur zwischen -40°C und +60°C in Diethylether, Tetrahydrofuran oder Dioxan mit einer metallorganischen Verbindung, bevorzugt mit einer aromatischen Grignard-Verbindung der allgemeinen Formel R³MgX (X = l, Br, Cl) oder einer Lithium-organischen Verbindung der allgemeinen Formel R³Li zu einer Verbindung der allgemeinen Formel III umgesetzt.

In der Verbindung der Formel III werden die Schutzgruppen mit Säure, bevorzugt mit wässriger Säure, besonders bevorzugt mit Salzsäure, ggf. in Gegenwart eines organischen Lösungsmittels wie z.B. Tetrahydrofuran oder Diehtylether bei einer Temperatur von -20°C bis +40°C, bevorzugt zwischen 0°C und Raumtemperatur abgespalten, und man erhält das Keton A.

Das solchermaßen erhaltene Keton A wird mit einem Tryptophol-, Benzofuran-3-ylethanol- oder Benzothiophen-3-ylethanol-Derivat, bevorzugt mit den entsprechenden Silylethern, besonders bevorzugt mit den entsprechenden Trimethylsilylethern in Gegenwart geeigneter Kupplungsreagentien, zu den Produkten la (X=O) umgesetzt. Als Kupplungsreagentien eignen sich starke Säuren wie z.B. Trifluoressigsäure, Methansulfonsäure oder ein Gemisch aus Phosphorsäure und Eisessig; oder Silylester besagter Säuren wie z.B. Trifluormethansulfonsäure-trimethylsilylester. Die Kupplungen erfolgen bei Temperaturen zwischen -10°C und +40°C, bevorzugt zwischen 0°C und Raumtemperatur, in einem organischen Lösungsmittel, bevorzugt Di- oder Trichlormethan oder Dichlorethan, oder man verwendet die Säure selbst als Lösungsmittel. Analog werden die entsprechenden Indol-3-ylethanthiol-, Benzofuran-3-ylethanthiol oder Benzothiophen-3-ylethanthiol-Derivate zu den Endprodukten la (X=S) umgesetzt.

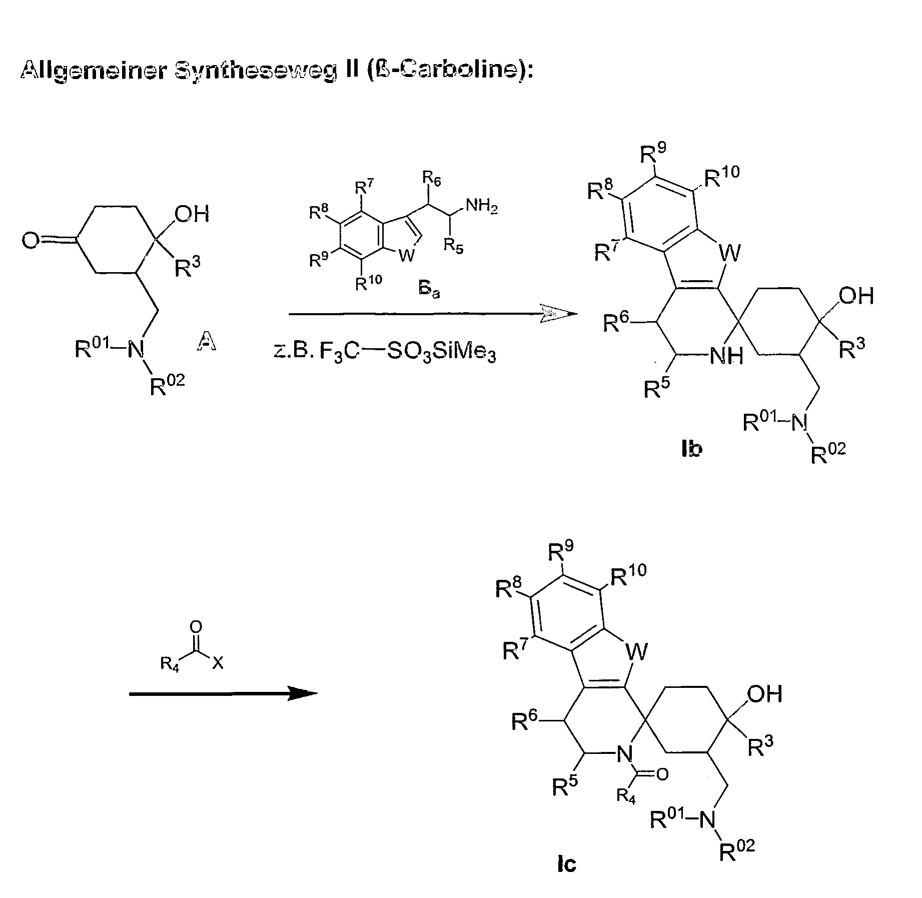

Das Keton A (siehe Allgemeines Syntheseschema I) wird mit einem Tryptamin-, Benzofuran-3-ylethylamin- oder Benzothiophen-3-ylethylamin-Derivat in Gegenwart geeigneter Kupplungsreagentien zu den Produkten Ib umgesetzt. Als Kupplungsreagentien eignen sich starke Säuren wie z.B. Trifluoressigsäure, Methansulfonsäure oder ein Gemisch aus Phosphorsäure und Eisessig; oder Silylester besagter Säuren wie z.B. Trifluormethansulfonsäure-trimethylsilylester. Die Kupplungen erfolgen bei Temperaturen zwischen -10°C und +40°C, bevorzugt zwischen 0°C und Raumtemperatur, in einem organischen Lösungsmittel, bevorzugt in einem Alkohol, besonders bevorzugt in Methanol, und anschließend in Di- oder Trichlormethan oder 1,2-Dichlorethan.

Die Produkte Ib können in einem Felgeschritt mit Säurehalogeniden oder - anhydriden in Gegenwart einer Base, beispielsweise Triethylamin, Pyridin oder (Dimethylamino)pyridin, zu den korrespondierenden Amiden Ic umgesetzt werden. Vorzugsweise findet diese Reaktion unter Mikroweiteneinstrahlung statt.

Spirocyclische Cyclohexanderivate der allgemeinen Formel I, bei denen X SO oder SO₂ bedeuten, können durch Umsetzung von spirocyclischen Cyclohexanderivaten der allgemeinen Formel I, bei denen X S bedeutet, mit einem Oxidationsmittel, beispielsweise H₂O₂, erhalten werden.

### Beispiele

Die folgenden Beispiele dienen zur näheren Erläuterung der Erfindung, schränken aber den allgemeinen Erfindungsgedanken nicht ein.

Die Ausbeuten der hergestellten Verbindungen sind nicht optimiert.

Alle Temperaturen sind unkorrigiert.

Die Angabe "Ether" bedeutet Diethylether, "EE" Ethylacetat und "DCM" Dichlormethan. Die Angabe "Äquivalente" bedeutet Stoffmengenäquivalente, "Smp." Schmelzpunkt bzw. Schmelzbereich, "Zers." Zersetzung, "RT" Raumtemperatur , "abs." absolut (wasserfrei), ,"rac." racemisch , "konz." konzentriert, "min" Minuten, "h" Stunden, "d" Tage, "Vol.%" Volumenprozent, "m%" Massenprozent und "M" ist eine Konzentrationsangabe in mol/l.

Als stationäre Phase für die Säulenchromatographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhälltnisse von Laufmitteln für chromatographische Untersuchungen sind stets in Volumen/Volumen angegeben.

Die im folgenden eingesetzten Verbindungen waren entweder kommerziell erhältlich, oder ihre Herstellung ist aus dem Stand der Technik bekannt oder in für den Fachmann offensichtlicher Weise aus dem Stand der Technik abgeleitet worden.

### 3-Dimethylaminomethyl-4-phenyl-4-hydroxy-cyclohexanon-hydrochlorid

Zu einer Lösung von 7-Dimethylaminomethyl-8-phenyl-1,4-dioxa-spiro[4.5]decan-8-ol-hydrochlorid (85 g, 259 mmol) in Tetrahydrofuran-Wasser-Gemisch wurden bei 0°C zügig 215 ml Salzsäure (32 %, Überschuss) gegeben und 16 Stunden bei RT nach rühren gelassen. Der ausgefallene, farblose Feststoff wurde abgesaugt und getrocknet. Es wurden 47,5 g (65%) des Produktes erhalten.

### 3-Dimethylaminomethyl-4-(3-fluorphenyl)-4-hydroxy-cyclohexanonmethansulfonat

Zu einer Lösung von 7-Dimethylaminomethyl-8-(3-fluorphenyl)-1,4-dioxaspiro[4.5]decan-8-ol-hydrochlorid (10,83 g, 31,3 mmol) in Tetrahydrofuran-Wasser-Gemisch wurden bei 0°C zügig 11,4 ml Salzsäure (32 %, Überschuss) gegeben und 16 Stunden bei RT nach rühren gelassen. Mit 32proz. Natronlauge wurde der Ansatz auf pH 11 gebracht und mit Ether extrahiert. Nach dem Verdampfen des Lösungsmittels im Vakuum wurde der Rückstand in Diisopropylether und EE aufgenommen und durch Zugabe von Methansulfonsäure (1,88 ml, 28,9 mmol) das entsprechende Salz gefällt. Es wurden 4,92 g (53%) des Produktes als farbloser Feststoff erhalten.

### 3-Dimethylaminomethyl-4-(2-methylphenyl)-4-hydroxy-cyclohexanonmethansulfonat

Zu einer Lösung von 7-Dimethylaminomethyl-8-(2-methyl-phenyl)-1,4-dioxaspiro[4.5]decan-8-ol-hydrochlorid (73,5 g, 215 mmol) in Tetrahydrofuran-Wasser-Gemisch wurden bei 0°C zügig 178 ml Salzsäure (32 %, Überschuss) gegeben und 16 Stunden bei RT nach rühren gelassen. Mit 32proz. Natronlauge wurde der Ansatz auf pH 12 gebracht und mit Ether extrahiert. Nach dem Verdampfen des Lösungsmittels im Vakuum wurde der Rückstand in Diisopropylether und EE aufgenommen und durch Zugabe von Methansulfonsäure (13 ml, 200 mmol) das entsprechende Salz gefällt. Es wurden 64,3 g (84%) des Produktes als farbloser Feststoff erhalten.

### 3-Dimethylaminomethyl-4-(4-trifluormethyl-phenyl)-4-hydroxy-cyclohexanonhydrochlorid

Zu einer Lösung von 7-Dimethylaminomethyl-8-(3-fluoro-phenyl)-1,4-dioxaspiro[4.5]decan-8-ol-hydrochlorid (35,9 g, 90,7 mmol) in Tetrahydrofuran-Wasser-Gemisch wurden bei 0°C zügig 60 ml Salzsäure (32 %, Überschuss) gegeben und 16 Stunden bei RT nach rühren gelassen. Mit 32proz. Natronlauge wurde der Ansatz auf pH 11 gebracht und mit Ether extrahiert. Nach dem Verdampfen des Lösungsmittels im Vakuum wurde der Rückstand in 200 ml Ethylmethylketon aufgenommen und durch Zugabe von Trimethylchlorsilan (11,2 ml) und Wasser (0,8 ml) das Hydrochlorid gefällt. Es wurden 23,5 g (74 %) des Produktes als farbloser Feststoff erhalten.

### 3-Dimethylaminomethyl-4-(4-methylthio-phenyl)-4-hydroxy-cyclohexanonhydrochlorid

Zu einer Lösung von 7-Dimethyfaminomethyl-8-(3-fluoro-phenyl)-1,4-dioxaspiro[4.5]decan-8-ol-hydrochlorid (41,0 g, 104 mmol) in Tetrahydrofuran-Wasser-Gemisch wurden bei 0°C zügig 70 ml Salzsäure (32 %, Überschuss) gegeben und 16 Stunden bei RT nach rühren gelassen. Der ausgefallene, farblose Feststoff wurde abgesaugt und getrocknet. Es wurden 20,8 g (57%) des Produktes erhalten.

### 2-Benzo[b]thiophen-3-yl-ethanol

Zu einer Suspension von Lithiumalanat (1,54 g, 41 mmol) in 35 ml Tetrahydrofuran wurde bei RT eine Lösung von Benzo[b]thiophen-3-yl-essigsäure (6,0 g, 31,2 mmol) in 40 ml Tetrahydrofuran getropft. Die Mischung wurde 60 Minuten lang bei 60°C gerührt, dann wurde mit Ethanol das überschüssige Alanat hydrolysiert. Es wurde über Kieselgur abgenutscht und das Lösungsmittel im Vakuum abgezogen. Man erhielt das Produkt als gelbliches Öl in einer Ausbeute von 5,1 g (92%).

### 2-Benzofuran-3-yl-ethanol

Zu einer Suspension von Lithiumalanat (1,61 g, 59,5 mmol) in 35 ml Ether wurde bei RT eine Lösung von Benzofuran-3-yl-essigsäure-methylester (4,0 g, 21 mmol) in 25 ml Ether getropft und 30 Minuten lang nach gerührt. Dann wurde mit Ethanol das überschüssige Alanat hydrolysiert, über Kieselgur abgenutscht und das Lösungsmittel im Vakuum abgezogen. Man erhielt das Produkt als gelbliches Öl in einer Ausbeute von 3,0 g (89%).

### Thioschwefelsäure-S-[2-(1H-indol-3-yl)-ethyl]-ester, O-Natriumsalz

Zu einer Lösung von Natrium-thiosulfat (6,0 g, 37,9 mmol) in 38 ml Wasser wurde bei RT eine Lösung von 3-(2-Brom-ethyl)-1 H-indol (5,0 g, 22,3 mmol) in 63 ml Ethanol getropft. Die Mischung wurde 30 Minuten lang refluxiert und danach das Lösungsmittel im Vakuum abdestilliert. Der Rückstand wurde in 110 ml Isopropanol suspendiert und refluxiert. Der ungelöste Rückstand wurde heiß abgesaugt, und aus dem Filtrat fiel beim Abkühlen das Produkt als farbloser Niederschlag aus (5,1 g, 82%).

### 2-(1H-Indol-3-yl)-ethanthiol

Thioschwefelsäure-S-[2-(1 H-indol-3-yl)-ethyl]-ester, O-Natriumsalz (5,04 g, 18 mmol) wurde in 105 ml 50proz. Phosphorsäure suspendiert und mit 160 ml Ether überschichtet. Der Ansatz wurde 6 Stunden lang refluxiert. Nach dem Abkühlen wurden die Phasen getrennt und die wässrige Phase mit Ether extrahiert und die organischen Phasen bis zur Trockene eingedampft. Man erhielt das Rohprodukt in einer Ausbeute von 3,2 g (100%).

### 1,1-(2-Dimethylaminomethyl)-3-hydroxy-3-(3-methoxyphenyl)pentamethylen)-3,4-dihydro-1H-2,9-diazafluoren

3-Dimethylaminomethyl-4-hydroxy-4-(3-methoxy-phenyl)-cyclohexanon (1,387 g, 5 mmol) und Tryptamin (800 mg, 5 mmol) wurden in 50ml trockenem 1,2-Dichlorethan gelöst. Unter Rühren wurden Trifluoressigsäure (770 µl, 10 mmol) und Natriumsulfat (2 g) hinzugefügt. Nach 15 Stunden wurde die Reaktionsmischung erneut mit 3ml Trifluoressigsäure versetzt und weitere 12 Stunden bei RT gerührt. Nach dem Einengen im Vakuum wurde der Rückstand mit Wasser (20 ml) versetzt, mit 5M NaOH auf pH 11 eingestellt und mit EE extrahiert. Nach dem Einengen wurde der Rückstand durch Chromatographie an Kieselgel mit EE/Ethanol (1 : 1 ) gereinigt und das Produkt in einer Ausbeute von 600 mg (29 %) als farbloser Feststoff erhalten.

### 1,1-(2-Dimethylaminomethyl)-3-hydroxy-3-(3-methoxyphenyl)pentamethylen)-3,4-dihydro-1H-2,9-diazafluoren-dihydrochlorid (Beispiel 1)

Zur Herstellung des Hydrochlorids wurde 1,1-(2-Dimethylaminomethyl)-3-hydroxy-3-(3-methoxyphenyl)pentamethylen)-3,4-dihydro-1H-2,9-diazafluoren (300 mg, 0,72 mmol) in Ethylmethylketon (5 ml) gelöst und mit Chlortrimethylsilan (237 µl, 1,79 mmol) versetzt. Der dabei entstandene Feststoff wurde abgesaugt und getrocknet. Das Hydrochlorid wurde in einer Ausbeute von 350 mg (100 %) als farbloser Feststoff gewonnen (Smp 236-238 °C).

### 1,1-(2-Dimethylaminomethyl)-3-hydroxy-3-(3-methoxyphenyl)pentamethylen)-2-acetyl-3,4-dihydro-1H-2,9-diazafluoren

1,1-(2-Dimethylaminomethyl)-3-hydroxy-3-(3-methoxyphenyl)pentamethylen)-3,4-dihydro-1 H-2,9-diazafluoren (300 mg, 0,72 mmol) wurde in Pyridin (5 ml) gelöst. Es wurde Acetanhydrid (674 µl, 7,2 mmol) zugetropft und 48 Stunden bei RT gerührt. Das Pyridin wurde im Vakuum abdestilliert, der Rückstand mit 10ml Wasser aufgenommen, mit 5M NaOH auf pH 11 eingestellt und mit EE extrahiert. Nach dem Einengen wurde das Produkt in einer Ausbeute von 323 mg (98 %) als farbloser Feststoff erhalten.

### 1,1-(2-Dimethylaminomethyl)-3-hydroxy-3-(3-methoxyphenyl)pentamethylen)-2-acetyl-3,4-dihydro-1H-2,9-diazafluoren-hydrochlorid (Beispiel 2)

Zur Herstellung des Hydrochlorids wurde 1,1-(2-Dimethylaminomethyl)-3-hydroxy-3-(3-methoxyphenyl)pentamethylen)-2-acetyl-3,4-dihydro-1 H-2,9-diazafluoren (323 mg, 0,7 mmol) in 5ml Ethylmethylketon gelöst und mit Chlortrimethylsilan (139 µl, 1,05 mmol) versetzt. Der dabei ausgefallene Feststoff wurde abgesaugt. Das Hydrochlorid wurde in einer Ausbeute von 348 mg (100 %) als farbloser Feststoff gewonnen (Smp 175-177 °C).

### 1,1-(2-(Dimethylaminomethyl)-3-hydroxy-3-(3-fluorphenyl)-pentamethylen)-3,4-dihydro-1H-2,9-diazafluoren

3-Dimethylaminomethyl-4-(3-fluorphenyl)-4-hydroxy-cyclohexanon (1,2 g, 4,5 mmol) und Tryptamin (0,72 g, 4,5 mmol) wurden in trockenem Methanol gelöst und mit Natriumsulfat (1,7 g) versetzt. Nach 24 Stunden wurde das Lösungsmittel, abdestilliert und der Rückstand in 36 ml 1,2-Dichlorethan suspendiert. Bei 0°C wurde Trifluoressigsäure (3,5 ml) zugetropft und 24 Stunden bei RT nach rühren gelassen. Dann wurde bei 0°C Wasser zugegeben und mit 5M Natronlauge auf pH 11 eingestellt. Die Phasen wurden getrennt und die wässrige Phase mit 1,2-Dichlorethan extrahiert. Nach dem Einengen wurde das Rohprodukt an Kieselgel chromatographiert (Eluens: Ether + 0,3% Ammoniak). Man erhielt 329 mg (0,8 mmol, 18%) des gewünschten Produktes.

### 1,1-(2-(Dimethylaminomethyl)-3-hydroxy-3-(3-fluorphenyl)-pentamethylen)-3,4-dihydro-1H-2,9-diazafluoren citrat (Beispiel 3)

1,1-(2-(Dimethylaminomethyl)-3-hydroxy-3-(3-fluorphenyl)-pentamethylen)-3,4-dihydro-1*H*-2,9-diazafluoren (329 mg, 0,8 mmol) wurden in 2 ml Ethanol gelöst und mit Citronensäure (155 mg, 0,8 mmol) versetzt. Das Citrat fiel als farbloser Feststoff aus (374 mg, 78%).

### 1,1-(2-(Dimethylaminomethyl)-3-hydroxy-3-phenyl-pentamethylen)-1,3,4,9-tetrahydro-pyrano[3,4-b]indol

3-Dimethylaminomethyl-4-phenyl-4-hydroxy-cyclohexanon (1 g, 4,0 mmol) und Tryptophol (0,65 g, 4,0 mmol) wurden in 40 ml Dichlormethan gelöst. Bei 0°C wurde Trifluormethansulfonsäure-trimethylsilylester zugetropft und 24 Stunden bei RT nach gerührt. Bei 0°C wurde mit Wasser hydrolysiert und mit 1 M Natronlauge auf pH 11 eingestellt. Die Phasen wurden getrennt und die wässrige Phase mit Dichlormethan extrahiert. Nach Einengen und Trocknen erhielt man 1,48 g (3,8 mmol, 95%) des Rohproduktes.

### 1,1-(2-(Dimethylaminomethyl)-3-hydroxy-3-phenyl-pentamethylen)-1,3,4,9-tetrahydro-pyrano[3,4-b]indol-sesquicitrat (Beispiel 4)

1,1-(2-(Dimethylaminomethyl)-3-hyd roxy-3-phenyl-pentamethylen)-1,3,4,9-tetrahydro-pyrano[3,4-b]indol (1,48 g, 3,8 mmol) wurde in 4 ml Ethanol gelöst und mit Citronensäure (730 mg, 3,8 mmol) versetzt. Dabei erhielt man 2,13 g (3,14 mmol, 83%) des Produktes.

### 1,1-(2-(Dimethylaminomethyl)-3-hydroxy-3-phenyl-pentamethylen)-1,3,4,9-tetrahydro-pyrano[3,4-b]benzofuran-citrat

3-Dimethylaminomethyl-4-phenyl-4-hydroxy-cyclohexanon (989 mg, 4,0 mmol) und 2-Benzofuran-3-yl-ethanol (649 mg, 4,0 mmol) wurden in 20 ml Dichlormethan gelöst. Bei RT wurde Trifluormethansulfonsäure-trimethylsilylester (0,8 ml, 4,14 mmol) zugetropft und 2 Stunden gerührt. Nach 30 Minuten Rühren mit 20 ml 1M Natronlauge wurden die Phasen getrennt und die wässrige Phase mit Dichlormethan extrahiert. Nach dem Einengen wurde das Rohprodukt an Kieselgel chromatographiert (Eluens: Dichlormethan/Methanol 19:1). Man erhielt das Produkt als gelben Schaum (1,1 g, 73%).

### 1,1-(2-(Dimethylaminomethyl)-3-hydroxy-3-phenyl-pentamethylen)-1,3,4,9-tetrahydro-pyrano[3,4-b]benzofuran-citrat (Beispiel 5)

1,1-(2-(Dimethylaminomethyl)-3-hydroxy-3-phenyl-pentamethylen)-1,3,4,9-tetrahydro-pyrano[3,4-b]benzofuran (1,1 g, 2,9 mmol) wurden in 10 ml Ethanol gelöst und mit Citronensäure (560 mg, 2,9 mmol) versetzt. Nach dem Einengen erhielt man das Produkt als hellgelben Schaum (1,6 g, 95%).

### 1,1-(2-(Dimethylaminomethyl)-3-hydroxy-3-(2-methylphenyl)-pentamethylen)-3,4-dihydro-1H-2,9-diazafluoren

3-Dimethylaminomethyl-4-(2-methylphenyl)-4-hydroxy-cyclohexanon (1,0 g, 3,8 mmol) und Tryptamin (0,61 g, 3,8 mmol) wurden in 30 ml trockenem Methanol gelöst. Nach Zugabe von Natriumsulfat (1,5 g) wurde noch 24 Stunden bei RT gerührt. Das Lösungsmittel wurde abdestilliert, der Rückstand in 30 ml 1,2-Dichlorethan suspendiert und mit 3 ml Trifluoressigsäure versetzt. Nach 24 Stunden Rühren bei RT wurde der Ansatz bei 0°C mit 25 ml Wasser versetzt und mit 5M Natronlauge auf pH 11 eingestellt. Es wurde 30 Minuten gerührt, die Phasen getrennt und mit 1,2-Dichlorethan extrahiert. Die Lösung wurde eingeengt und mit Ether aufgenommen. Dabei wurde das Rohprodukt als farbloser Feststoff erhalten (338 mg, 22%).

### 1,1-(2-(Dimethylaminomethyl)-3-hydroxy-3-(2-methylphenyl)-pentamethylen)-3,4-dihydro-1H-2,9-diazafluoren citrat (Beispiel 6)

1,1-(2-(Dimethlaminomethyl)-3-hydroxy-3-(2-methylphenyl)-pentamethylen)-3,4-dihydro-1*H*-2,9-diazafluoren (338 mg, 0,84 mmol) wurden in 8 ml Ethanol gelöst und mit Citronensäure (161 mg, 0,84 mmol) versetzt. Das Produkt fiel als farbloser Niederschlag (290 mg, 58%) aus.

### 1,1-(2-(Dimethylaminomethyl)-3-hydroxy-3-phenyl-pentamethylen)-1,3,4,9-tetrahydro-pyrano[3,4-b]benzothiophen

3-Dimethylaminomethyl-4-phenyl-4-hydrol-cyclohexanon (989 mg, 4,0 mmol) und 2-Benzo[b]thiophen-3-yl-ethanol (713 mg, 4,0 mmol) wurden in 20 ml Dichlormethan gelöst. Bei RT wurde Trifluormethansulfonsäure-trimethylsilylester (0,8 ml, 4,14 mmol) zugetropft und 2 Stunden nach gerührt. Dann wurden 30 Minuten mit 20 ml 1M Natronlauge gerührt. Die Phasen wurden getrennt und die wässrige Phase mit Dichlormethan extrahiert. Als Rohprodukt erhielt man einen farblosen Schaum (1,53 g, 94%).

### 1,1-(2-(Dimethylaminomethyl)-3-hydroxy-3-phenyl-pentamethylen)-1,3,4,9-tetrahydro-pyrano[3,4-b]benzothiophen-citrat (Beispiel 7)

1,1-(2-(Dimethylaminomethyl)-3-hydroxy-3-phenyl-pentamethylen)-1,3,4,9-tetrahydro-pyrano[3,4-b]benzothiophen (1,53 g, 3,8 mmol) wurden in 10 ml Ethanol gelöst und mit 748 mg Citronensäure versetzt. Nach 16 Stunden wurde eingeengt und ein fast farbloser Schaum als Produkt isoliert (2,0 g, 88%).

### 1,1-(2-(Dimethylaminomethyl)-3-hydroxy-3-(3-fluorphenyl)-pentamethylen)-1,3,4,9-tetrahydro-pyrano[3,4-b]benzofuran

3-Dimethylaminomethyl-4-(3-fluorphenyl)-4-hydroxy-cyclohexanon (796 mg, 3,0 mmol) und 2-Benzofuran-3-yl-ethanol (487 mg, 3,0 mmol) wurden in Dichlormethan gelöst und bei RT mit Trifluormethansulfonsäure-trimethylsilylester (0,59 ml, 3,06 mmol) versetzt. Es wurde 90 Minuten bei RT gerührt, dann wurden 15 ml 1M Natronlauge zugegeben und weitere 30 Minuten gerührt. Die Phasen wurden getrennt und die wässrige Phase mit Dichlormethan extrahiert. Nach Eindampfen des Lösungsmittels wurde das Rohprodukt in einer Ausbeute von 1,2 g (95%) erhalten.

### 1,1-(2-(Dimethylaminomethyl)-3-hydroxy-3-(3-fluorphenyl)-pentamethylen)-1,3,4,9-tetrahydro-pyrano[3,4-b]benzofuran-citrat (Beispiel 8)

1,1-(2-(Dimethylaminomethyl)-3-hydroxy-3-(3-fluorphenyl)-pentamethylen)-1,3,4,9-tetrahydro-pyrano[3,4-b]benzofuran (1,2 g, 1,9 mmol) wurden in 8 ml Ethanol gelöst und mit 563 mg Citronensäure versetzt. Nach 18 Stunden Rühren bei RT wurde die Lösung eingeengt. Man erhielt das Produkt als farblosen Schaum (1,7 g, 2,8 mmol, 97%).

### 1,1-(2-(Dimethylaminomethyl)-3-hydroxy-3-phenyl-pentamethylen)-1,3,4,9-tetrahydro-2-thia-9-aza-fluoren

3-Dimethylaminomethyl-4-phenyl-4-hydroxy-cyclohexanon (1,4 g, 5,7 mmol) und 2-(1 H-Indol-3-yl)-ethanthiol (1,0 g, 5,7 mmol) wurden in 26 ml Eisessig gelöst. Bei ca. 10°C wurden 6 ml Phosphorsäure zugetropft und 24 Stunden bei RT gerührt. Bei 0°C wurden 190 ml 1M Natronlauge und 150 ml Dichlormethan zugesetzt und 30 Minuten bei RT gerührt. Die Phasen wurden getrennt und die wässrige Phase mit Dichlormethan exrahiert. Nach Einengen des Lösungsmittels wurden 1,35 g (59%) des Rohproduktes erhalten.

### 1,1-(2-(Dimethylaminomethyl)-3-hydroxy-3-phenyl-pentamethylen)-1,3,4,9-tetrahydro-2-thia-9-aza-fluoren-citrat (Beispiel 9)

1,1-(2-(Dimethylaminomethyl)-3-hydroxy-3-phenyl-pentamethylen)-1,3,4,9-tetrahydro-2-thia-9-aza-fluoren (1,35 g, 3,3 mmol) wurden in 4 ml Ethanol gelöst und mit Citronensäure (640 mg, 3,3 mmol) versetzt. Beim Einengen wurde das Produkt als farbloser Schaum erhalten (1,82 g, 92%).

### 1,1-(2-(Dimethylaminomethyl)-3-hydroxy-3-(3-fluorphenyl)-pentamethylen)-1,3,4,9-tetrahydro-pyrano[3,4-b]benzothiophen

3-Dimethylaminomethyl-4-(3-fluorphenyl)-4-hydroxy-cyclohexanon (796 mg (3,0 mmol) und 2-Benzo[b]thiophen-3-yl-ethanol (535 mg, 3,0 mmol) wurden in Dichlormethan gelöst und bei RT mit Trifluormethansulfonsäure-trimethylsilylester (0,59 ml, 3,06 mmol) versetzt. Nach 2 Stunden bei RT wurden 15 ml 1M Natronlauge zugegeben und 30 Minuten gerührt. Die Phasen wurden getrennt und mit Dichlormethan extrahiert. Nach dem Einengen wurde an Kieselgel chromatographiert (Eluens: Dichlormethan / Methanol 19:1), und man erhielt das Produkt als farblosen Schaum (700 mg, 57%).

### 1,1-(2-(Dimethylaminomethyl)-3-hydroxy-3-(3-fluorphenyl)-pentamethylen)-1,3,4,9-tetrahydro-pyrano[3,4-b]benzothiophen-citrat (Beispiel 10)

1,1-(2-(Dimethylaminomethyl)-3-hydroxy-3-(3-fluorphenyl)-pentamethylen)-1,3,4,9-tetrahydro-pyrano[3,4-b]benzothiophen (700 mg, 1,7 mmol) wurden in 5 ml Ethanol gelöst und mit Citronensäure (326 mg) versetzt. Es wurde 16 Stunden bei RT gerührt. Beim Einengen erhielt man einen farblosen Schaum (1,05 g, 99%).

### 1,1-(2-(Dimethylaminomethyl)-3-hydroxy-3-(2-methylphenyl)-pentamethylen)-1,3,4,9-tetrahydro-pyrano[3,4-b]benzothiophen

3-Dimethylaminomethyl-4-(2-methylphenyl)-4-hydroxy-cycloxanon (653 mg, 2,5 mmol) und 2-Benzo[b]thiophen-3-yl-ethanol (450 mg, 2,5 mmol) wurden in Dichlormethan gelöst und bei RT mit Trifluormethansulfonsäure-trimethylsilylester (0,48 ml, 2,6 mmol) versetzt. Nach 2 Stunden Rühren bei RT wurde mit 15 ml 1M Natronlauge versetzt und weitere 30 Minuten gerührt. Die Phasen wurden getrennt und die wässrige Phase mit Dichlormethan extrahiert. Das Rohprodukt wurde an Kieselgel chromatographiert (Eluens: Dichlormethan / Methanol 9:1). Man erhielt das gewünschte Produkt als gelblichen Feststoff (900 mg, 85%).

### 1,1-(2-(Dimethylaminomethyl)-3-hydroxy-3-(2-methylphenyl)-pentamethylen)-1,3,4,9-tetrahydro-pyrano[3,4-b]benzothiophen-citrat (Beispiel 11)

1,1-(2-(Dimethylaminomethyl)-3-hydroxy-3-(2-methylphenyl)-pentamethylen)-1,3,4,9-tetrahydro-pyrano[3,4-b]benzothiophen (900 mg, 2,13 mmol) wurden in 5 ml Ethanol gelöst, mit Citronensäure (410 mg) versetzt und 16 Stunden bei RT gerührt. Nach dem Einengen erhielt man einen farblosen Feststoff (1,2 g, 92%).

### 1,1-(2-(Dimethylaminomethyl)-3-hydroxy-3-(2-methylphenyl)-pentamethylen)-1,3,4,9-tetrahydro-pyrano[3,4-b]benzofuran

3-Dimethylaminomethyl-4-(2-methylphenyl)-4-hydroxy-cyclohexanon (784 mg, 3,0 mmol) und 2-Benzofuran-3-yl-ethanol (487 mg, 3,0 mmol) wurden in Dichlormethan gelöst und bei RT mit Trifluormethansulfonsäure-trimethylsilylester (0,59 ml, 3,06 mmol) versetzt. Nach 2 Stunden Rühren bei RT wurde mit 15 ml 1M Natronlauge versetzt und weitere 30 Minuten gerührt. Die Phasen wurden getrennt und die wässrige Phase mit Dichlorethan extrahiert. Das Rohprodukt wurde an Kieselgel chromatographiert (Eluens: Dichlormethan / Methanol 9:1). Man erhielt das gewünschte Produkt als gelblichen Schaum (760 mg, 62%).

### 1,1-(2-(Dimethylaminomethyl)-3-hydroxy-3-(2-methylphenyl)-pentamethylen)-1,3,4,9-tetrahydro-pyrano[3,4-b]bensofuran-citrat (Beispiel 12)

1,1-(2-(Dimethylaminomethyl)-3-hydroxy-3-(2-methylphenyl)-pentamethylen)-1,3,4,9-tetrahydro-pyrano[3,4-b]benzofuran (760 mg, 1,9 mmol) wurden in 5 ml Ethanol gelöst, mit Citronensäure (360 mg) versetzt und 16 Stunden bei RT gerührt. Nach dem Einengen erhielt man einen farblosen Schaum (1,0 g, 88%).

### 1,1-(2-(Dimethylaminomethyl)-3-hydroxy-3-(4-trifluormethylphenyl)-penta-methylen)-1,3,4,9-tetrahydro-pyrano[3,4-b]benzofuran

3-Dimethylaminomethyl-4-(4-trifluormethylphenyl)-4-hydroxy-cyclohexanon (946 mg, 3,0 mmol) und 2-Benzofuran-3-yl-ethanol (487 mg, 3,0 mmol) wurden in Dichlormethan gelöst und bei RT mit Trifluormethansulfonsäure-trimethylsilylester (0,59 ml, 3,06 mmol) versetzt. Nach 2 Stunden Rühren bei RT wurde mit 15 ml 1M Natronlauge versetzt und weitere 30 Minuten gerührt. Die Phasen wurden getrennt und die wässrige Phase mit Dichlormethan extrahiert. Das Rohprodukt wurde an Kieselgel chromatographiert (Eluens: Dichlormethan / Methanol 9:1). Man erhielt das gewünschte Produkt als gelblichen Schaum (560 mg, 41%).

### 1,1-(2-(Dimethylaminomethyl)-3-hydroxy-3-(4-trifluormethylphenyl)-pentamethylen)-1,3,4,9-tetrahydro-pyrano[3,4-b]benzofuran-citrat (Beispiel 13)

1,1-(2-(Dimethylaminomethyl)-3-hydroxy-3-(4-trifluormethylphenyl)-pentamethylen)-1,3,4,9-tetrahydro-pyrano[3,4-b]benzofuran (560 mg, 1,22 mmol) wurden in 5 ml Ethanol gelöst, mit Citronensäure (234 mg) versetzt und 16 Stunden bei RT gerührt. Nach dem Einengen erhielt man einen farblosen Schaum (785 mg, 99%).

### 1,1-(2-(Dimethylaminomethyl)-3-hydroxy-3-(3-fluorphenyl)-pentamethylen)-1,3,4,9-tetrahydro-2-thia-9-aza-fluoren

3-Dimethylaminomethyl-4-(3-fluorphenyl)-4-hydroxy-cyclohexanon (1,0 g, 3,8 mmol) und 2-(1 H-Indol-3-yl)-ethanthiol (670 mg, 3,8 mmol) wurden in 17 ml Eisessig gelöst.

Bei 55°C wurden 4 ml Phosphorsäure zugetropft und 24 Stunden bei RT gerührt. Bei 5°C wurden 130 ml 1M Natronlauge und 100 ml Dichlormethan zugesetzt und 2 Stunden bei RT gerührt. Die Phasen wurden getrennt und die wässrige Phase mit Dichlormethan extrahiert. Nach Einengen des Lösungsmittels wurden 839 mg (52%) des Rohproduktes erhalten.

### 1,1-(2-(Dimethylaminomethyl)-3-hydroxy-3-(3-fluorphenyl)-pentamethylen)-1,3,4,9-tetrahydro-2-thia-9-aza-fluoren-citrat (Beispiel 14)

1,1-(2-(Dimethylaminomethyl)-3-hydroxy-3-(3-fluorphenyl)-pentamethylen)-1,3,4,9-tetrahydro-2-thia-9-aza-fluoren (839 mg, 1,98 mmol) wurden in 3 ml Ethanol gelöst und mit Citronensäure (380 mg, 2,0 mmol) versetzt. Das Produkt wurde als farbloser Feststoff erhalten (1,26 g, 97%).

### 1,1-(2-(Dimethylaminomethyl)-3-hydroxy-3-(4-trifluormethylphenyl)pentamethylen)-3,4-dihydro-1H-2,9-diazafluoren

3-Dimethylaminomethyl-4-(4-trifluormethyl-phenyl)-4-hydroxy-cyclohexanon (1,26 g, 4,0 mmol) und Tryptamin (640 mg, 4,0 mmol) wurden in 26 ml Methanol gelöst, mit 1,28 g Natriumsulfat versetzt und 2 Stunden bei RT gerührt. Nach Abdestillieren des Lösungsmittels wurde der Rückstand in 26 ml 1,2-Dichlorethan aufgenommen. Bei 0°C wurde Trifluoressigsäure (3,2 ml) zugegeben und 24 Stunden bei RT gerührt. Bei 0°C wurden 26 ml Wasser zugegeben und mit 5M Natronlauge auf pH 11 eingestellt. Nach Phasentrennung wurde die wässrige Phase mit 1,2-Dichlorethan extrahiert. Man erhielt 364 mg (22%) des Rohproduktes.

### 1,1-(2-(Dimethylaminomethyl)-3-hydroxy-3-(4-trifluormethylphenyl)pentamethylen)-3,4-dihydro-1H-2,9-diazafluoren dicitrat (Beispiel 15)

1,1-(2-(Dimethylaminomethyl)-3-hydroxy-3-(4-trifluormethylphenyl)-pentamethylen)-3,4-dihydro-1*H*-2,9-diazafluoren (364 mg, 0,9 mmol) wurden in 4 ml Ethanol gelöst und mit Citronensäure (166 mg, 0,9 mmol) versetzt. Das Produkt wurde als farbloser Niederschlag (356 mg, 47%) erhalten.

### 1,1-(2-(Dimethylaminomethyl)-3-hydroxy-3-(3-fluorphenyl)-pentamethylen)-7-fluor-3,4-dihydro-1H-2,9-diazafluoren

3-Dimethylaminomethyl-4-(3-fluor-phenyl)-4-hydroxy-cyclohexanon (1,1g, 4,1 mmol) und Tryptamin (740 mg, 4,1 mmol) wurden in 33 ml Methanol gelöst, mit 1,64 g Natriumsulfat versetzt und 1 Stunde bei RT gerührt. Nach Abdestillieren des Lösungsmittels wurde der Rückstand in 30 ml 1,2-Dichlorethan aufgenommen. Bei 0°C wurde Trifluoressigsäure (3,28 ml) zugegeben und 24 Stunden bei RT gerührt. Bei 0°C wurden 30 ml Wasser zugegeben und mit 5M Natronlauge auf pH 11 eingestellt. Nach Phasentrennung wurde die wässrige Phase mit 1,2-Dichlorethan exrahiert. Das Rohprodukt wurde an Kieselgel (Eluens: EE / Methanol 4:1) chromatographiert, und man erhielt das Produkt in einer ausbeute von 89 mg (5%).

### 1,1-(2-(Dimethylaminomethyl)-3-hydroxy-3-(3-fluorphenyl)-pentamethylen)-7-fluor-3,4-dihydro-1H-2,9-diazafluoren-sesquicitrat (Beispiel 16)

1,1-(2-(Dimethylaminomethyl)-3-hydroxy-3-(3-fluorphenyl)-pentamethylen)-7-fluor-3,4-dihydro-1*H*-2,9-diazafluoren (89 mg, 0,2 mmol) wurden in 3 ml Ethanol gelöst und mit Citronensäure (40 mg, 0,21 mmol) versetzt. Das Produkt wurde als farbloser Niederschlag (66 mg, 46%) erhalten.

### 1,1-(2-(Dimethylaminomethyl)-3-hydroxy-3-phenyl-pentamethylen)-3,4-dihydro-6-methoxy-1H-2,9-diazafluoren

3-Dimethylaminomethyl-4-phenyl-4-hydroxy-cyclohexanon (980 mg, 4,0 mmol) und Tryptamin (740 mg, 4,1 mmol) wurden in 33 ml Methanol gelöst, mit 1,6 g Natriumsulfat versetzt und 1 Stunde bei RT gerührt. Nach Abdestillieren des Lösungsmittels wurde der Rückstand in 30 ml 1,2-Dichlorethan aufgenommen. Bei 0°C wurde Trifluoressigsäure (3,2 ml) zugegeben und 24 Stunden bei RT gerührt. Bei 0°C wurden 30 ml Wasser zugegeben und mit 5M Natronlauge auf pH 11 eingestellt. Nach Phasentrennung wurde die wässrige Phase mit 1,2-Dichlorethan extrahiert. Das Rohprodukt wurde an Kieselgel (Eluens: Ether mit 1 % 25proz Ammoniak-Lösung) chromatographiert, und man erhielt das Produkt in einer Ausbeute von 575 mg (33%).

### 1,1-(2-(Dimethylaminomethyl)-3-hydroxy-3-phenyl-pentamethylen)-3,4-dihydro-6-methoxy-1H-2,9-diazafluoren citrat (Beispiel 17)

1,1-(2-(Dimethylaminomethyl)-3-hydroxy-3-phenyl-pentamethylen)-3,4-dihydro-6-methoxy-1*H*-2,9-diazafluoren (575 mg, 1,3 mmol) wurden in 10 ml Ethanol gelöst und mit Citronensäure (255 mg, 1,33 mmol) versetzt. Das Produkt wurde als farbloser Niederschlag (586 mg, 96%) erhalten.

### 1,1-(2-(Dimethylaminomethyl)-3-hydroxy-3-(4-methylthiophenyl)-pentamethylen)-1,3,4,9-tetrahydro-3-methoxy-pyrano[3,4-b]indol

3-Dimethylaminomethyl-4-(4-methylthiophenyl)-4-hydroxy-cyclohexanon (733 mg, 2,5 mmol) und 5-Methoxy-tryptamin (475 mg, 2,5 mmol) wurden in 20 ml Methanol gelöst, mit 1,0 g (7 mmol) Natriumsulfat versetzt und 1 Stunde bei RT gerührt. Nach Abdestillieren des Lösungsmittels wurde der Rückstand in 20 ml 1,2-Dichlorethan aufgenommen. Bei 0°C wurde Trifluoressigsäure (2,0 ml, 26 mmol) zugegeben und 24 Stunden bei RT gerührt. Es wurden 20 ml Wasser zugegeben und mit 5M Natronlauge auf pH 11 eingestellt. Nach Phasentrennung wurde die wässrige Phase mit 1,2-Dichlorethan extrahiert. Das Rohprodukt wurde an Kieselgel (Eluens: Dichlormethan / Methanol 9:1) chromatographiert, und man erhielt das Produkt in einer Ausbeute von 740 mg (63%).

### 1,1-(2-(Dimethylaminomethyl)-3-hydroxy-3-(4-methylthiophenyl)-pentamethylen)-1,3,4,9-tetrahydro-6-methoxy-pyrano[3,4-b]indol-citrat (Beispiel 18)

1,1-(2-(Dimethylaminomethyl)-3-hydroxy-3-(4-methylthiophenyl)-pentamethylen)-1,3,4,9-tetrahydro-6-methoxy-pyrano[3,4-b]indol (520 mg, 1,11 mmol) wurden in 15 ml Ethanol gelöst und mit Citronensäure (219 mg) versetzt. Das Produkt wurde als farbloser Niederschlag (670 mg, 92%) erhalten.

### 1,1-(2-(Dimethylaminomethyl)-3-hydroxy-3-(2-methylphenyl)-pentamethylen)-1,3,4,9-tetrahydro-6-methoxy-pyrano[3,4-b]indol

3-Dimethylaminomethyl-4-(2-methylphenyl)-4-hydroxy-cyclohexanon (653 mg, 2,5 mmol) und 5-Methoxy-tryptamin (475 mg, 2,5 mmol) wurden in 20 ml Methanol gelöst, mit 1,0 g (7 mmol) Natriumsulfat versetzt und 1 Stunde bei RT gerührt. Nach Abdestillieren des Lösungsmittels wurde der Rückstand in 20 ml 1,2-Dichlorethan aufgenommen. Bei RT wurde Trifluoressigsäure (2,0 ml, 26 mmol) zugegeben und 24 Stunden bei RT gerührt. Es wurden 20 ml Wasser zugegeben und mit 5M Natronlauge auf pH 11 eingestellt. Nach Phasentrennung wurde die wässrige Phase mit 1,2-Dichlorethan extrahiert. Das Rohprodukt wurde an Kieselgel (Eluens: Dichlormethan / Methanol 19:1) chromatographiert, und man erhielt das Produkt in einer Ausbeute von 800 mg (74%).

### 1,1-(2-(Dimethylaminomethyl)-3-hydroxy-3-(2-methylphenyl)-pentamethylen)-1,3,4,9-tetrahydro-6-methoxy-pyrano[3,4-b]indol-citrat (Beispiel 19)

1,1-(2-(Dimethylaminomethyl)-3-hydroxy-3-(2-methylphenyl)-pentamethylen)-1,3,4,9-tetrahydro-6-methoxy-pyrano[3,4-b]indol (550 mg, 1,27 mmol) wurden in 5 ml Ethanol gelöst und mit Citronensäure (235 mg) versetzt. Das Produkt wurde als farbloser Niederschlag (580 mg, 73%) erhalten.

### 1,1-(2-(Dimethylaminomethyl)-3-hydroxy-3-(4-methylthiophenyl)-pentamethylen)-1,3,4,9-tetrahydro-pyrano-[3,4-b]indol

3-Dimethylaminomethyl-4-(4-methylthio-phenyl)-4-hydroxy-cyclohexanon (1,2 g, 4,1 mmol) und Tryptophol (660 mg, 4,1 mmol) wurden in 40 ml Dichlormethan gelöst und bei 0°C mit Trifluormethansulfonsäure-trimethylsilylester (3,69 ml, 20,4 mmol) versetzt. Es wurde 24 Stunden bei RT gerührt, dann wurde mit 30 ml Wasser versetzt und 1M Natronlauge auf pH11 eingestellt. Die Phasen wurden getrennt und die wässrige Phase mit Dichlormethan extrahiert. Das Rohprodukt wurde an Kieselgel (Eluens: EE / Methanol 9:1) chromatographiert. Man erhielt 270 mg (0,62 mmol, 15%) des Produktes.

### 1,1-(2-(Dimethylaminomethyl)-3-hydroxy-3-(4-methylthiophenyl)-pentamethylen)-1,3,4,9-tetrahydro-pyrano[3,4-b]indol-citrat (Beispiel 20)

1,1-(2-(Dimethylaminomethyl)-3-hydroxy-3-(4-methylthiophenyl)-pentamethylen)-1,3,4,9-tetrahydro-pyrano[3,4-b]indol (270 mg, 0,62 mmol) wurden in 10 ml Ethanol suspendiert und mit Citronensäure (119 mg, 0,62 mmol) versetzt. Nach Eindampfen des Lösungsmittels erhielt man das Produkt als farblosen Schaum (411 mg, 65%).

### 1,1-(2-(Dimethylaminomethyl)-3-hydroxy-3-(3-fluorphenyl)-pentamethylen)-3,4-dihydro-6-methoxy-1H-2,9-diazafluoren

3-Dimethylaminomethyl-4-(3-fluorphenyl)-4-hydroxy-cyclohexanon (663 mg, 2,5 mmol) und 5-Methoxytryptamin (475 mg, 2,5 mmol) wurden in 20 ml Methanol gelöst, mit 1,0 g Natriumsulfat versetzt und 1 Stunde bei RT gerührt. Nach dem Abdestillieren des Lösungsmittels wurde der Rückstand mit 20 ml 1,2-Dichlorethan suspendiert, bei RT mit Trifluoressigsäure (2,0 ml, 26 mmol) versetzt und 3 Stunden gerührt. Dann wurde der Ansatz mit 20 ml Wasser versetzt, mit Natronlauge auf pH 11 eingestellt und die wässrige Phase mit 1,2-Dichlorethan extrahiert. Das Rohprodukt wurde an Kieselgel chromatographiert (Eluens: Ether mit 2% 25proz. Ammoniak). Man erhielt 395 mg (36%) des Produktes.

### 1,1-(2-(Dimethylaminomethyl)-3-hydroxy-3-(3-fluorphenyl)-pentamethylen)-3,4-dihydro-6-methoxy-1H-2,9-diazafluoren citrat (Beispiel 21)

1,1-(2-(Dimethylaminomethyl)-3-hydroxy-3-(3-fluorphenyl)-pentamethylen)-3,4-dihydro-6-methoxy-1*H*-2,9-diazafluoren (395 mg, 0,9 mmol) wurden bei 70°C in 5 ml Ethanol gelöst und mit Citronensäure (173 mg) versetzt. Der nach dem Abkühlen erhielt man das Produkt als farblosen Niederschlag (315 mg, 56%).

### 1,1-(2-(Dimethylaminomethyl)-3-hydroxy-3-(4-trifluormethylphenyl)-pentamethylen)-3,4-dihydro-6-methoxy-1H-2,9-diazafluoren

3-Dimethylaminomethyl-4-(4-trifluormethylphenyl)-4-hydroxy-cyclohexanon (788 mg, 2,5 mmol) und 5-Methoxytryptamin (475 mg, 2,5 mmol) wurden in 20 ml Methanol gelöst, mit 1,0 g Natriumsulfat versetzt und 3 Stunden bei RT gerührt. Nach dem Abdestillieren des Lösungsmittels wurde der Rückstand mit 20 ml 1,2-Dichlorethan suspendiert, bei RT mit Trifluoressigsäure (2,0 ml, 26 mmol) versetzt und 3 Stunden gerührt. Dann wurde der Ansatz mit 20 ml Wasser versetzt, mit Natronlauge auf pH 11 eingestellt und die wässrige Phase mit 1,2-Dichlorethan extrahiert. Das Rohprodukt wurde an Kieselgel chromatographiert (Eluens: Ether mit 2% 25proz. Ammoniak). Man erhielt 717 mg (66%) des Produktes.

### 1,1-(2-(Dimethylaminomethyl)-3-hydroxy-3-(4-trifluormethylphenyl)-pentamethylen)-3,4-dihydro-6-methoxy-1H-2,9-diazafluoren citrat (Beispiel 22)

1,1-(2-(Dimethylaminomethyl)-3-hyd roxy-3-(4-trifluormethylphenyl)-pentamethylen)-3,4-dihydro-6-methoxy-1*H*-2,9-diazafluoren (717 mg, 1,47 mmol) wurden bei 50°C in 5 ml Ethanol gelöst und mit Citronensäure (282 mg) versetzt. Nach dem Abkühlen erhielt man das Produkt als farblosen Niederschlag (860 mg, 86%).

### 1,1-(2-(Dimethylaminomethyl)-3-hydroxy-3-(4-methylthiophenyl)-pentamethylen)-1,3,4,9-tetrahydro-2-thia-9-aza-fluoren

3-Dimethylaminomethyl-4-(4-methylthio-phenyl)-4-hydroxy-cyclohexon (1,0 g, 3,4. mmol) und 2-(1H-Indol-3-yl)-ethanthiol (604 mg, 3,4 mmol) wurden in 15 ml Eisessig gelöst. Bei 10°C wurden 4 ml 85proz. Phosphorsäure zugetropft und 20 Stunden bei RT gerührt. Bei 10°G wurden 96 ml Dichlormethan zugegeben, mit 5M Natronlage auf pH 9 eingestellt und 1 Stunde nach gerührt. Die Phasen wurden getrennt und die wässrige Phase mit Dichlormethan extrahiert. Nach Abdestillieren des Lösungsmittels erhielt man das Rohprodukt in einer Ausbeute von 964 mg (63%).

### 1,1-(2-(Dimethylaminomethyl)-3-hydroxy-3-(4-methylthiophenyl)-pentamethylen)-1,3,4,9-tetrahydro-2-thia-9-aza-fluoren-sesquicitrat (Beispiel 23)

1,1-(2-(Dimethylaminomethyl)-3-hydroxy-3-(4-methylthiophenyl)-pentamethylen)-1,3,4,9-tetrahydro-2-thia-9-aza-fluoren (710 mg, 1,57 mmol) wurde in der Siedehitze in 10 ml Ethanol gelöst. Dazu wurde Citronensäure (1,57 mmol) gegeben. Nach dem Einengen erhielt man das Produkt als gelblichen Schaum in einer Ausbeute von 779 mg (67%).

### 1,1-(2-(Dimethylaminomethyl)-3-hydroxy-3-(2-methylphenyl)-pentamethylen)1,3,4,9-tetrahydro-2-thia-9-aza-fluoren

3-Dimethylaminomethyl-4-(2-methylphenyl)-4-hydroxy-cyclohexanon (980 mg, 3,7 mmol) und 2-(1H-Indol-3-yl)-ethanthiol (660 mg, 3,7 mmol) wurden in 17 ml Eisessig gelöst. Bei 5°C wurden 4 ml 85proz. Phosphorsäure zugetropft und 20 Stunden bei RT gerührt. Bei 5°C wurden 100 ml Dichlormethan zugegeben, mit 5M Natronlauge auf pH 9 eingestellt und 1 Stunde nach gerührt. Die Phasen wurden getrennt und die wässrige Phase mit Dichlormethan extrahiert. Nach Abdestillieren des Lösungsmittels erhielt man das Rohprodukt in einer Ausbeute von 541 mg (35%).

### 1,1-(2-(Dimethylaminomethyl)-3-hydroxy-3-(2-methylphenyl)-pentamethylen)-1,3,4,9-tetrahydro-2-thia-9-aza-fluoren-citrat (Beispiel 24)

1,1-(2-(Dimethylaminomethyl)-3-hydroxy-3-(2-methylphenyl)-pentamethylen)-1,3,4,9-tetrahydro-2-thia-9-aza-fluoren (541 mg, 1,29 mmol) wurde in 5 ml Ethanol gelöst und mit Citronensäure (219 mg) versetzt. Nach dem Einengen erhielt man das Produkt als bräunlichen Schaum in einer Ausbeute von 677 mg (86%).

### 1,1-(Dimethylaminomethyl)-3-hydroxy-3-(2-methylphenyl)-pentamethylen)-1,3,4,9-tetrahydro-pyrano[3,4-b]indol

3-Dimethylaminomethyl-4-(2-methylphenyl)-4-hydroxy-cyclohexanon (1,0 g, 3,8 mmol) und Tryptophol (620 mg, 3,8 mmol) wurden in 38 ml Dichlormethan gelöst. Bei 0°C wurde Trifluormethansulfonsäure-trimethylsilylester (3,46 ml, 19 mmol) zugegeben und 24 Stunden bei RT gerührt. Unter Eiskühlung wurde mit 30 ml Wasser versetzt und mit 1M Natronlauge auf pH 11 eingestellt. Die Phasen wurden getrennt und mit Dichlormethan extrahiert. Das Rohprodukt wurde an Kieselgel chromatographiert (Eluens: EE / Hexan 1:2 + 1% 25proz. Ammoniak). Man erhielt das Produkt in einer Ausbeute von 190 mg (12%).

### 1,1-(2-(Dimethylaminomethyl)-3-hydroxy-3-(2-methylphenyl)-pentamethylen)-1,3,4,9-tetrahydro-pyrano[3,4-b]indol-citrat (Beispiel 25)

1,1-(2-(Dimethylaminomethyl)-3-hydroxy-3-(2-methylphenyl)-pentamethylen)-1,3,4,9-tetrahydro-pyrano[3,4-b]indol (190 mg, 0,47 mmol) wurde in 4 ml Ethanol gelöst und mit 90 mg Citronensäure (0,47 mmol) versetzt. Nach dem Einengen erhielt man 280 mg (0,469 mmol, 99%) des Produktes.

### 1,1-(2-(Dimethylaminomethyl)-3-hydroxy-3-(4-methylthio-phenyl)-pentamethylen)-3,4-dihydro-1H-2,9-diazafluoren

3-Dimethylaminomethyl-4-(4-methylthio-phenyl)-4-hydroxy-cyclohexanon (180 mg, 0,6 mmol) und Tryptamin (100 mg, 0,6 mmol) wurden in 10 ml Methanol gelöst, mit 240 mg Natriumsulfat versetzt und 24 Stunden bei RT gerührt. Nach dem Abdestillieren des Lösungsmittels wurde der Rückstand mit 10 ml 1,2-Dichlorethan suspendiert, bei RT mit Trifluoressigsäure (0,48 ml) versetzt und 24 Stunden gerührt. Dann wurde der Ansatz mit 5 ml Wasser versetzt, mit Natronlauge auf pH 11 eingestellt und die wässrige Phase mit 1,2-Dichlorethan extrahiert. Das Rohprodukt wurde an Kieselgel chromatographiert (Eluens: Ether mit 0,3% 25proz. Ammoniak). Man erhielt 74 mg (28%) des Produktes.

### 1,1-(2-(Dimethylaminomethyl)-3-hydroxy-3-(4-methylthiophenyl)-pentamethylen)-3,4-dihydro-1H-2,9-diazafluoren citrat (Beispiel 26)

1,1-(2-(Dimethylaminomethyl)-3-hydroxy-3-(4-methylthiophenyl)-pentamethylen)-3,4-dihydro-1*H*-2,9-diazafluoren (74 mg, 0,17 mmol) wurden in 2 ml Ethanol gelöst und mit Citronensäure (38 mg, 0,2 mmol) versetzt. Das Produkt fiel als farbloser Niederschlag aus (56 mg, 53%).

### 1,1-(2-(Dimethylaminomethyl)-3-hydroxy-3-(3-fluorphenyl)-pentamethylen)-1,3,4,9-tetrahydro-pyrano[3,4-b]indol

3-Dimethylaminomethyl-4-(3-fluorphenyl)-4-hydroxy-cyclohexanon (490 mg, 1,9 mmol) und Tryptophol (300 mmol, 1,9 mmol) wurden in 17 ml Dichlormethan gelöst. Bei RT wurde Methansulfonsäure (0,24 ml, 3,7 mmol) zugetropft und 24 Stunden gerührt. Mit 1M Natronlauge wurde auf pH 11 eingestellt, die Phasen getrennt und die wässrige Phase mit Dichlormethan extrahiert. Man erhielt das Rohprodukt in einer Ausbeute von 801 mg (107%).

### 1,1-(2-(Dimethylaminomethyl)-3-hydroxy-3-(3-fluorphenyl)-pentamethylen)-1,3,4,9-tetrahydro-pyrano[3,4-b]indol-hydrochlorid (Beispiel 27)

1,1-(2-(Dimethylaminomethyl)-3-hydroxy-3-(3-fluorphenyl)-pentamethylen)-1,3,4,9-tetrahydro-pyrano[3,4-b]indol (801 mg, 2,0 mmol) wurde in Ethylmethylketon gelöst und mit 19 µl Wasser und 273 µl Trimethylchlorsilan versetzt. Nach Zugabe von Ether fiel das Produkt als gräulicher Niederschlag aus (124 mg, 15%).

### 1,1-(2-(Dimethylaminomethyl)-3-hydroxy-3-phenyl-pentamethylen)-3,4-dihydro-1H-2,9-diazafluoren

3-Dimethylaminomethyl-4-phenyl-4-hydroxy-cyclohexanon (1,0 g, 4,0 mmol) und Tryptamin (650 mg, 4,0 mmol) wurden in 35 ml Methanol gelöst, mit 1,8 g Natriumsulfat versetzt und 24 Stunden bei RT gerührt. Nach dem Abdestillieren des Lösungsmittels wurde der Rückstand in 35 ml 1,2-Dichlorethan suspendiert, bei RT mit Trifluoressigsäure (3,2 ml) versetzt und 24 Stunden gerührt. Dann wurde der Ansatz mit 30 ml Wasser versetzt, mit Natronlauge auf pH 11 eingestellt und die wässrige Phase mit 1,2-Dichlorethan extrahiert. Das Rohprodukt wurde an Kieselgel chromatographiert (Eluens: EE mit 1% 25proz. Ammoniak). Beim Aufnehmen in EE blieb ein Teil des Produktes als Niederschlag zurück. Man erhielt so 80 mg (5%) des Produktes.

### 1,1-(2-(Dimethylaminomethyl)-3-hydroxy-3-phenyl-pentamethylen)-3,4-dihydro-1H-2,9-diazafluoren citrat (Beispiel 28)

1,1-(2-(Dimethylaminomethyl)-3-hydroxy-3-phenyl-pentamethylen)-3,4-dihydro-1H-2,9-diazafluoren (80 mg, 0,2 mmol) wurden in 3 ml Ethanol gelöst und mit Citronensäure (38 mg, 0,2 mmol) versetzt. Das Produkt fiel als farbloser Niederschlag aus (104 mg, 91%).

Die Beispiele 29-31 wurden analog Beispiel 7 und die Beispiele 32-35 analog Beispiel 6 hergestellt.

| **Beispiele-nummer** | **Struktur (als, Base)** | **Name** |
|---|---|---|
| **1** | 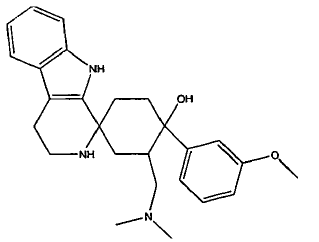 | 1,1-(2-(Dimethylaminomethyl)-3-hydroxy-3-(3-methoxyphenyl)pentamethylen)-3,4-dihydro-1H-2,9-diazafluoren-dihydrochlorid |
| **2** | 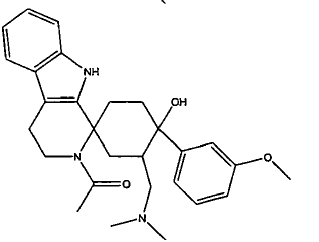 | 1,1-(2-(Dimethylaminomethyl)-3-hydroxy-3-(3-methoxyphenyl)pentamethylen)-2-acetyl-3,4-dihydro-1H-2,9-diazafluoren-hydrochlorid |
| **3** | 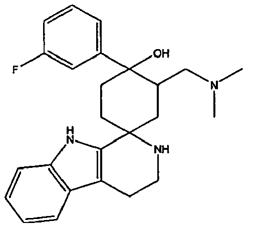 | 1,1-(2-(Dimethylaminomethyl)-3-hydroxy-3-(3-fluorphenyl)-pentamethylen)-3,4-dihydro-1*H*-2,9-diazafluoren citrat |
| **4** | 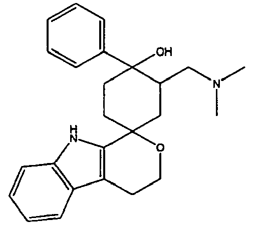 | 1,1-(2-(Dimethylaminomethyl)-3-hydroxy-3-phenyl-pentamethylen)-1,3,4,9-tetrahydro-pyrano[3,4-b]indol-sesquicitrat |
| **5** | 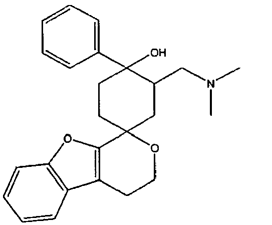 | 1,1-(2-(Dimethylaminomethyl)-3-hydroxy-3-phenyl-pentamethylen)-1,3,4,9-tetrahydro-pyrano[3,4-b]benzofuran-citrat |
| **6** | 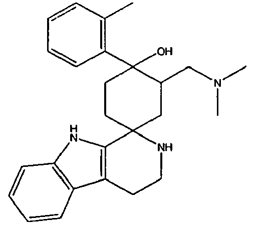 | 1,1-(2-(Dimethylaminomethyl)-3-hydroxy-3-(2-methylphenyl)-pentamethylen)-3,4-dihydro-1*H*-2,9-diazafluorene citrat |
| **7** | 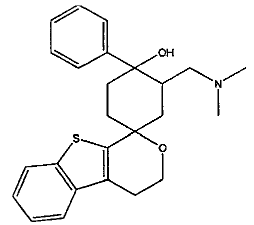 | 1,1-(2-(Dimethylaminomethyl)-3-hydroxy-3-phenyl-pentamethylen)-1,3,4,9-tetrahydro-pyrano[3,4-b]benzothiophen-citrat |
| **8** | 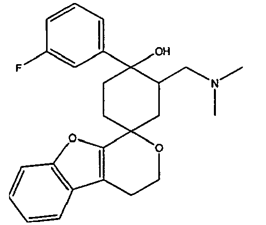 | 1,1-(2-(Dimethylaminomethyl)-3-hydroxy-3-(3-fluorphenyl)-pentamethylen)-1,3,4,9-tetrahydro-pyrano[3,4-b]benzofuran-citrat |
| **9** | 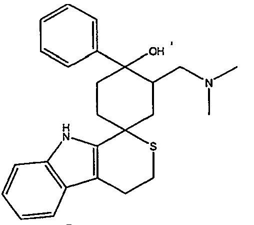 | 1,1-(2-(Dimethylaminomethyl)-3-hydroxy-3-phenyl-pentamethylen)-1,3,4,9-tetrahydro-2-thia-9-aza-fluoren-citrat |
| **10** | 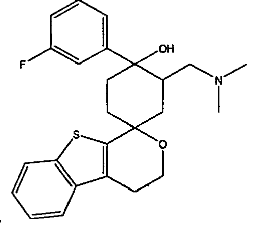 | 1,1-(2-(Dimethylaminomethyl)-3-hydroxy-3-(3-fluorphenyl)-pentamethylen)-1,3,4,9-tetrahydro-pyrano[3,4-b]benzothiophen-citrat |
| **11** | 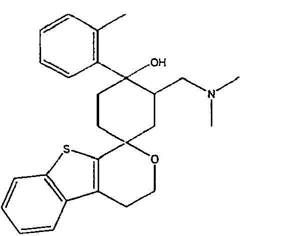 | 1,1-(2-(Dimethylaminomethyl)-3-hydroxy-3-(2-methylphenyl)-pentamethylen)-1,3,4,9-tetrahydro-pyrano[3,4-b]benzothiophen-citrat |
| **12** | 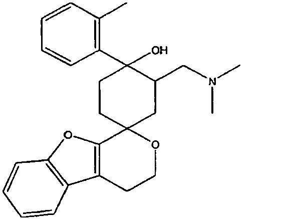 | 1,1-(2-(Dimethylaminomethyl)-3-hydroxy-3-(2-methylphenyl)-pentamethylen)-1,3,4,9-tetrahydro-pyrano[3,4-b]benzofuran-citrat |
| **13** | 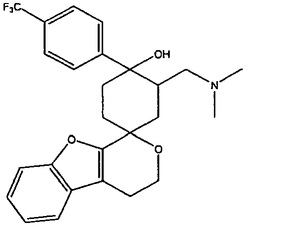 | 1,1-(2-(Dimethylaminomethyl)-3-hydroxy-3-(4-trifluormethylphenyl)-pentamethylen)-1,3,4,9-tetrahydro-pyrano[3,4-b]benzofuran-citrat |
| **14** | 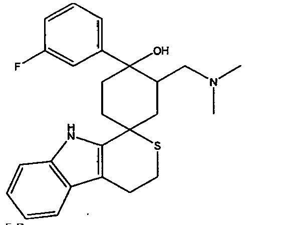 | 1,1-(2-(Dimethylaminomethyl)-3-hydroxy-3-(3-fluorphenyl)-pentamethylen)-1,3,4,9-tetrahydro-2-thia-9-aza-fluoren-citrat |
| **15** | 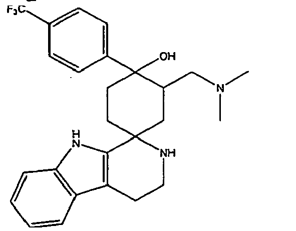 | 1,1-(2-(Dimethylaminomethyl)-3-hydroxy-3-(4-trifluormethylphenyl)-pentamethylen)-3,4-dihydro-1*H*-2,9-diazafluoren dicitrat |
| **16** | 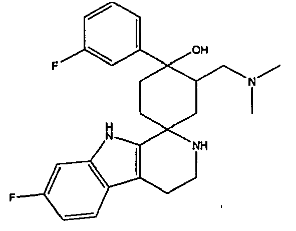 | 1,1-(2-(Dimethylaminomethyl)-3-hydroxy-3-(3-fluorphenyl)-pentamethylen)-7-fluor-3,4-dihydro-1*H*-2,9-diazafluoren-sesquicitrat |
| **17** | 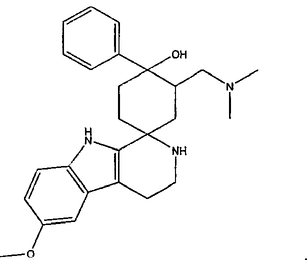 | 1,1-(2-(Dimethylaminomethyl)-3-hydroxy-3-phenyl-pentamethylen)-3,4-dihydro-6-methoxy-1*H*-2,9-diazafluoren citrat |
| **18** | 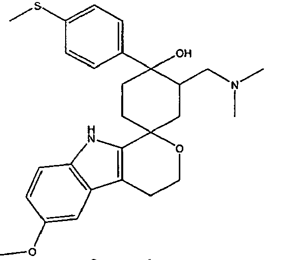 | 1,1-(2-(Dimethylaminomethyl)-3-hydroxy-3-(4-methylthiophenyl)-pentamethylen)-1,3,4,9-tetrahydro-6-methoxy-pyrano[3,4-b]indol-citrat |
| **19** | 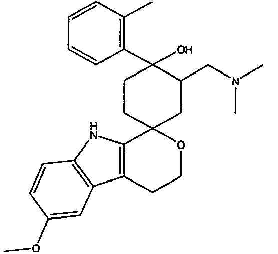 | 1,1-(2-(Dimethylaminomethyl)-3-hydroxy-3-(2-methylphenyl)-pentamethylen)-1,3,4,9-tetrahydro-6-methoxy-pyrano[3,4-b]indol-citrat |
| **20** | 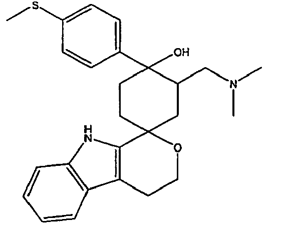 | 1,1-(2-(Dimethylaminomethyl)-3-hydroxy-3-(4-methylthiophenyl)-pentamethylen)-1,3,4,9-tetrahydro-pyrano[3,4-b]indol-citrat |
| **21** | 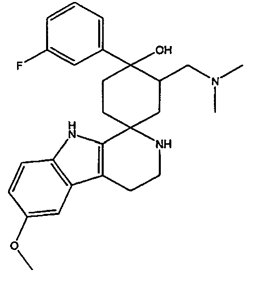 | 1,1-(2-(Dimethylaminomethyl)-3-hydroxy-3-(3-fluorphenyl)-pentamethylen)-3,4-dihydro-6-methoxy-1*H*-2,9-diazafluoren citrat |
| **22** | 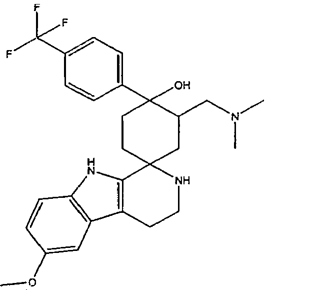 | 1,1-(2-(Dimethylaminomethyl)-3-hydroxy-3-(4-trifluormethylphenyl)-pentamethylen)-3,4-dihydro-6-methoxy-1*H*-2,9-diazafluoren citrat |
| **23** | 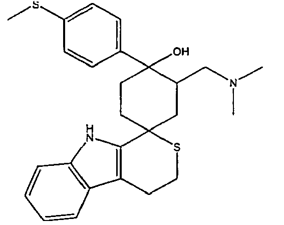 | 1,1-(2-(Dimethylaminomethyl)-3-hydroxy-3-(4-methylthiophenyl)-pentamethylen)-1,3,4,9-tetrahydro-2-thia-9-aza-fluoren-sesquicitrat |
| **24** | 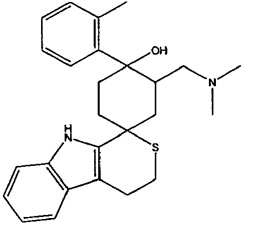 | 1,1-(2-(Dimethylaminomethyl)-3-hydroxy-3-(2-methylphenyl)-pentamethylen)-1,3,4,9-tetrahydro-2-thia-9-aza-fluoren-citrat |
| **25** | 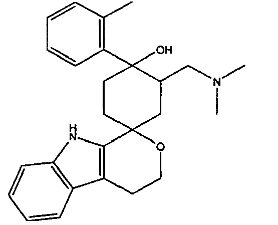 | 1,1-(2-(Dimethylaminomethyl)-3-hydroxy-3-(2-methylphenyl)-pentamethylen)-1,3,4,9-tetrahydro-pyrano[3,4-b]indol-citrat |
| **26** | 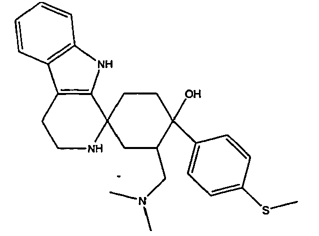 | 1,1-(2-(Dimethylaminomethyl)-3-hydroxy-3-(4-methylthiophenyl)-pentamethylen)-3,4-dihydro-1*H*-2,9-diazafluoren citrat |
| **27** | 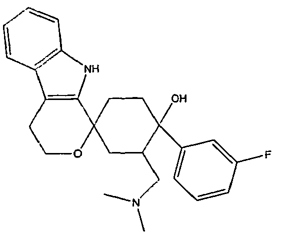 | 1,1-(2-(Dimethylaminomethyl)-3-hydroxy-3-(3-fluorphenyl)-pentamethylen)-1,3,4,9-tetrahydro-pyrano[3,4-b]indol-hydrochlorid |
| **28** | 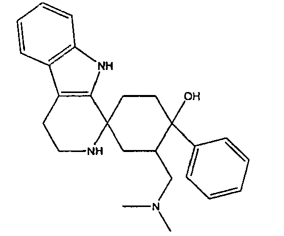 | 1,1-(2-(Dimethylaminomethyl)-3-hydroxy-3-pheny)-pentamethylen)-3,4-dihydro-1H-2,9-diazafluoren citrat |
| **29** | 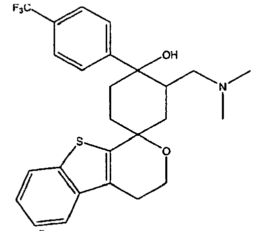 | 1,1-(2-(Dimethylaminomethyl)-3-hydroxy-3-(4-trifluormethylphenyl)-pentamethylen)-1,3,4,9-tetrahydro-pyrano[3,4-b]benzothiophen-citrat |
| **30** | 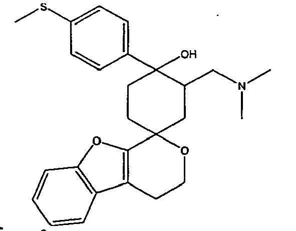 | 1,1-(2-(Dimethylaminomethyl)-3-hydroxy-3-(4-methylthiophenyl)-pentamethylen)-1,3,4,9-tetrahydro-pyrano[3,4-b]benzofuran-citrat |
| **31** | 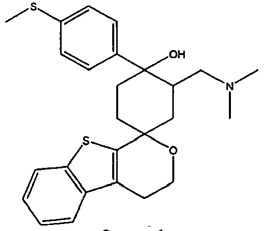 | 1,1-(2-(Dimethylaminomethyl)-3-hydroxy-3-(4-methylthiophenyl)-pentamethylen)-1,3,4,9-tetrahydro-pyrano[3,4-b]benzothiophen-citrat |
| **32** | 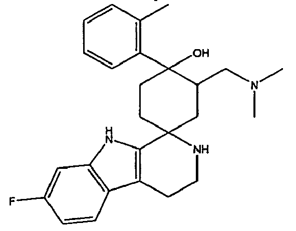 | 1,1-(2-(Dimethylaminomethyl)-3-hydroxy-3-(2-methylphenyl)-pentamethylen)-7-fluor-3,4-dihydro-1*H*-2,9-diazafluoren citrat |
| **33** | 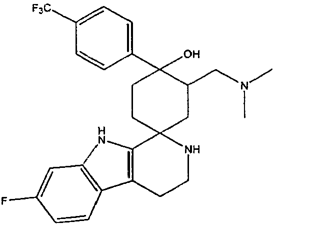 | 1,1-(2-(Dimethylaminomethyl)-3-hydroxy-3-(4-trifluormethylphenyl)-pentamethylen)-7-fluor-3,4-dihydro-1*H*-2,9-diazafluoren citrat |
| **34** | 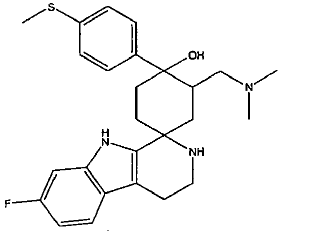 | 1,1-(2-(Dimethylaminomethyl)-3-hydroxy-3-(4-methylthiophenyl)-pentamethylen)-7-fluor-3,4-dihydro-1*H*-2,9-diazafluoren citrat |
| **35** | 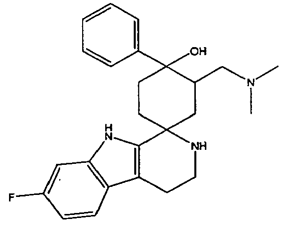 | 1,1-(2-(Dimethylaminomethyl)-3-hydroxy-3-phenyl-pentamethylen)-7-fluor-3,4-dihydro-1*H*-2,9-diazafluoren dicitrat |

### Biologische Testung

### a) Untersuchungen zur Serotonin-Wiederaufnahmehemmung (5HT-Uptake-Hemmung)

Um diese in vitro Studien durchführen zu können, werden Synaptosomen aus Rattenhirnarealen frisch isoliert. Es findet jeweils eine sogenannte "P₂"-Fraktion Verwendung, die nach der Vorschrift von Gray und Whittaker (E.G. Gray und V.P. Whittaker (1962) J. Anat. 76, 79-88) präpariert wird. Für den 5HT-Uptake werden diese vesikulären Partikel aus dem Hypothalamus männlicher Rattengehirne isoliert.

Eine detaillierte Methodenbeschreibung kann der Literatur entnommen werden (M.Ch. Frink, H.-H. Hennies, W. Englberger, M. Haurand und B. Wilffert (1996) Arzneim.-Forsch./Drug Res. 46 (III), 11, 1029-1036).

### b) Untersuchungen zur Noradrena/in-Wiederaufnahmehemmung (NA-Uptake-Hemmung)

Um diese in vitro Studien durchführen zu können, werden Synaptosomen aus Rattenhirnarealen frisch isoliert. Es findet jeweils eine sogenannte "P₂"-Fraktion Verwendung, die nach der Vorschrift von Gray und Whittaker (E.G. Gray und V.P. Whittaker (1962) J. Anat. 76, 79-88) präpariert wird. Für den NA-Uptake werden diese vesikulären Partikel aus dem Hypothalamus männlicher Rattengehirne isoliert.

Eine detaillierte Methodenbeschreibung kann der Literatur entnommen werden (M.Ch. Frink, H.-H. Hennies, W. Englberger, M. Haurand und B. Wilffert (1996) Arzneim.-Forsch./Drug Res. 46 (III), 11, 1029-1036).

### c) Messung der µ-Bindung

Die Rezeptoraffinität zum humanen µ-Opiatrezeptor wurde in einem homogenen Ansatz in Mikrotiterplatten bestimmt. Hierzu wurden Verdünnungsreihen des jeweils zu prüfenden substituierten spirocyclischen Cyclohexan-Derivates mit einer Rezeptormembranpräparation (15-40 µg Protein pro 250 µl Inkubationsansatz) von CHO-K1-Zellen, welche den humanen µ-Opiatrezeptor exprimieren (RB-HOM-Rezeptormembran-Präparation der Firma NEN, Zaventem, Belgien) in Gegenwart von 1 nmol/l des radioaktiven Liganden [³H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 µmol/l Naloxon zugegeben. Nach Beendigung der neunzigminütigen Inkubationszeit wurden die Mikrotiterplatten für 20 Minuten bei 1000 g abzentrifugiert und die Radioaktivität in einem β-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen µ-Opiatrezeptor bei einer Konzentration der Prüfsubstanzen von 1 µmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I IC₅₀ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten.

Beispielhaft wurden die folgenden Bindungsdaten bestimmt:

| **Beispiele-nummer** | **µ-Bindung (%) [1 µM]** |
|---|---|
| **1** | 40 |
| **2** | 65 |
| **4** | 74 |
| **5** | 58 |
| **8** | 73 |
| **12** | 56 |
| **27** | 64 |
| **28** | 57 |
| **34** | 54 |

| **Beispiel-nummer** | **5-HT-Uptake, %Hemmung (10 µM)** |
|---|---|
| **1** | 44 |
| **3** | 75 |
| **4** | 84 |
| **5** | 84 |
| **6** | 69 |
| **7** | 88 |
| **8** | 88 |
| **9** | 81 |
| **10** | 88 |
| **11** | 85 |
| **12** | 68 |
| **13** | 60 |
| **14** | 61 |
| **15** | 83 |
| **16** | 72 |
| **17** | 57 |
| **20** | 58 |
| **21** | 57 |
| **22** | 47 |
| **23** | 50 |
| **24** | 75 |
| **27** | 83 |
| **29** | 47 |
| **30** | 83 |
| **31** | 82 |
| **32** | 75 |
| **33** | 78 |
| **34** | 65 |
| **35** | 67 |

| **Beispiel-nummern** | **NA-Uptake, %Hemmung (10 µM)** |
|---|---|
| **1** | 43 |
| **3** | 48 |
| **5** | 51 |
| **7** | 59 |
| **8** | 66 |
| **10** | 55 |
| **11** | 43 |
| **12** | 41 |
| **13** | 46 |
| **14** | 46 |
| **15** | 76 |
| **18** | 41 |
| **21** | 44 |
| **24** | 42 |
| **26** | 40 |
| **29** | 35 |
| **30** | 37 |
| **31** | 60 |
| **32** | 38 |
| **33** | 68 |
| **34** | 57 |

### Parenterale Lösung eines erfindungsgemäßen spirocyclischen Cyclohexan-Derivats

38 g eines der erfindungsgemäßen spirocyclischen Cyclohexan-Derivate, hier Beispiel 1, wird in 1 l Wasser für Injektionszwecke bei Raumtemperatur gelöst und anschließend durch Zugabe von wasserfreier Glukose für Injektionszwecke auf isotone Bedingungen eingestellt.

## Patentansprüche

1. Spirocyclische Cyclohexan-Derivate der allgemeinen Formel I, 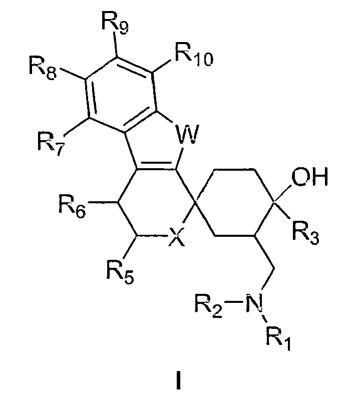 , worin
R¹ und R², unabhängig voneinander für H; CHO; C₁₋₅-Alkyl jeweils gesättigt oder ungesättigt, verzweigt oder unverzweigt, einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, stehen;
oder die Reste R¹ und R² zusammen für CH₂CH₂OCH₂CH₂, CH₂CH₂NR¹¹CH₂CH₂ oder (CH₂)₃₋₆ stehen,
wobei R¹¹ H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, bedeutet;
R³ für Phenyl, Phenethyl, Thiophenyl, Pyridyl oder Benzyl, jeweils unsubstituiert oder einfach oder mehrfach substituiert, steht;
W für NR⁴, O oder S steht
und
R⁴ für H; C₁₋₅-Alkyl, gesättigt oder ungesättigt, verzweigt oder unverweigt, unsubstituiert oder einfach oder mehrfach substituiert; Aryl, oder Heteroaryl, jeweils substituiert oder unsubstituiert; über eine C₁₋₃-Alkyl-Gruppe gebundenes Aryl, Heteroaryl oder Cycloalkyl, jeweils einfach oder mehrfach substituiert oder unsubstituiert; COR¹² ; SO₂R¹² steht,
wobei R¹² H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert; OR¹³; NR¹⁴R¹⁵ bedeutet;
R⁵ für =O; H; COOR¹³, CONR¹³, OR¹³; C₁₋₅-Alkyl, gesättigt oder ungesättigt, verzweigt oder unverzweigt, unsubstituiert oder einfach oder mehrfach substituiert; C₃₋₈-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, steht;
R⁶ für H; F, Cl, NO₂, CF₃, OR¹³, SR¹³, SO₂R¹³, SO₂OR¹³, CN, COOR¹³, NR¹⁴R¹⁵; C₁₋₅₋Alkyl, gesättigt oder ungesättigt, verzweigt oder unverzweigt, unsubstituiert oder einfach oder mehrfach substituiert; C₃₋₈-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Akyl gebundenes Aryl, C₃₋₈-Cycloalkyl oder Heteroaryl, unsubstituiert oder einfach oder mehrfach substituiert, steht;
oder R⁵ und R⁶ gemeinsam (CH₂)ₙ mit n = 2, 3, 4, 5 oder 6 bedeuten, wobei einzelne Wasserstoffatome auch durch F, Cl, Br, I, NO₂, CF₃, OR¹³, CN oder C₁₋₅-Alkyl ersetzt sein können;
R⁷, R⁸, R⁹ und R¹⁰ unabhängig voneinander für H, F, Cl, Br, I, NO₂, CF₃, OR¹³, SR¹³, SO₂R¹³, SO₂OR¹³, CN, COOR¹³, NR¹⁴R¹⁵; C₁₋₅-Alkyl, C₃₋₈-Cycloalkyl, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, stehen;
wobei R¹³ H; C₁₋₅-Alkyl jeweils gesättigt oder ungesättigt, verzweigt oder unverzweigt, unsubstituiert oder einfach oder mehrfach substituiert; C₃₋₈-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, bedeutet;
R¹⁴ und R¹⁵ unabhängig voneinander H; C₁₋₅-Alkyl , jeweils gesättigt oder ungesättigt, verzweigt oder unverzweigt, unsubstituiert oder einfach oder mehrfach substituiert; oder C₃₋₈ -Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, bedeuten;
oder R¹⁴ und R¹⁵ zusammen CH₂CH₂OCH₂CH₂, CH₂CH₂NR¹⁶CH₂CH₂ oder (CH₂)₃₋₆ bilden,
wobei R¹⁶ H; C₁₋₅-Alkyl gesättigt oder ungesättigt, verzweigt oder unverzweigt, unsubstituiert oder einfach oder mehrfach substituiert, bedeutet;
X für O, S, SO, SO₂ oder NR¹⁷ steht;
R¹⁷ für H; C₁₋₅-Alkyl, gesättigt oder ungesättigt, verzweigt oder unverzweigt; COR¹² oder SO₂R¹² steht,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

2. Spirocyclische Cyclohexan-Derivate gemäß Anspruch 1, **dadurch gekennzeichnet, dass**
R¹ und R² unabhängig voneinander für H; C₁₋₅-―Alkyl, gesättigt oder ungesättigt, verzweigt oder unverzweigt, einfach oder mehrfach substituiert oder unsubstituiert, stehen;
oder die Reste R¹ und R² zusammen einen Ring bilden und CH₂CH₂OCH₂CH₂, CH₂CH₂NR¹¹CH₂CH₂ oder (CH₂)₃₋₆ bedeuten,
wobei R¹¹ H; C₁₋₅-Alkyl, gesättigt oder ungesättigt, verzweigt oder unverzweigt, einfach oder mehrfach substituiert oder unsubstituiert, bedeutet.

3. Spirocyclische Cyclohexan-Derivate gemäß Anspruch 1, **dadurch gekennzeichnet, dass** dass R¹ und R² unabhängig voneinander für CH₃ oder H stehen, wobei R¹ und R² nicht gleichzeitig H bedeuten.

4. Spirocyclische Cyclohexan-Derivate gemäß, einem der Ansprüche 1 bis 3, **dadurch gekennzeichnet, dass** R³ Phenyl, unsubstituiert oder einfach oder mehrfach substituiert, bedeutet.

5. Spirocyclische Cyclohexan-Derivate gemäß, einem der Ansprüche 1 bis 4, **dadurch gekennzeichnet, dass** R³ Phenyl bedeutet, unsubstituiert oder mit F, Cl, CN, OCH₃, SCH₃, OCH₂CH₃, CH₃, CF₃ oder OH einfach oder mehrfach substituiert, insbesondere Phenyl, 3-Methoxyphenyl, 2-Methylphenyl, 4-Trifluormethylphenyl, 4-Methylthiophenyl, 3-Fluorphenyl und 4-Fluorphenyl.

6. Spirocyclische Cyclohexan-Derivate gemäß einem der Ansprüche 1 bis 5, **dadurch gekennzeichnet, dass** W für NR⁴, O oder S und X für NR¹⁷, O oder S steht

7. Spirocyclische Cyclohexan-Derivate gemäß einem der Ansprüche 1 bis 5, **dadurch gekennzeichnet, dass** W für NH, O oder S und X für NH, NC(O)CH₃, O oder S steht

8. Spirocyclische Cyclohexan-Derivate gemäß einem der Ansprüche 1 bis 7, **dadurch gekennzeichnet, dass** R⁷, R⁸, R⁹ und R¹⁰ unabhängig voneinander für H; C₁₋₅-Alkyl, verzweigt oder unverzweigt, unsubstituiert oder ein- oder mehrfach substituiert; F, Cl, Br, I, CF₃, OH, OCH₃, NH₂, COOH, COOCH₃, NHCH₃ oder N(CH₃)₂ oder NO₂ stehen, insbesondere H, F oder OCH₃.

9. Spirocyclische Cyclohexan-Derivate gemäß einem der Ansprüche 1 bis 8, **dadurch gekennzeichnet, dass** R⁵ für H, C₁₋₅-Alkyl, verzweigt oder unverzweigt, unsubstituiert oder einfach oder mehrfach substituiert oder COOR¹³ steht und R⁶ für H oder C₁₋₅-Alkyl steht, insbesondere R⁵ und R⁶ H bedeuten.

10. Spirocyclische Cyclohexan-Derivate gemäß einem der Ansprüche 1 bis 7, **dadurch gekennzeichnet, dass** die Reste R⁵-R¹⁰ H bedeuten.

11. Spirocyclische Cyclohexan-Derivate gemäß einem der Ansprüche 1 bis 10 aus der Gruppe
1,1-(2-(Dimethylaminomethyl)-3-hydroxy-3-(3-methoxyphenyl)pentamethylen)-3,4-dihydro-1H-2,9-diazafluoren-dihydrochlorid
1,1-(2-(Dimethylaminomethyl)-3-hydroxy-3-(3-methoxyphenyl)pentamethylen)-2-acetyl-3,4-dihydro-1H-2,9-diazafluoren-hydrochlorid
1,1-(2-(Dimethylaminomethyl)-3-hydroxy-3-(3-fluorphenyl)-pentamethylen)-3,4-dihydro-1*H*-2,9-diazafluoren citrat
1,1-(2-(Dimethylaminomethyl)-3-hydroxy-3-phenyl-pentamethylen)-1,3,4,9-tetrahydro-pyrano[3,4-b]indol-sesquicitrat
1,1-(2-(Dimethylaminomethyl)-3-hyd roxy-3-phenyl-pentamethylen)-1,3,4,9-tetrahydro-pyrano[3,4-b]benzofuran-citrat
1,1-(2-(Dimethylaminomethyl)-3-hydroxy-3-(2-methylphenyl)-pentamethylen)-3,4-dihydro-1*H*-2,9-diazafluoren citrat
1, 1-(2-(Dimethylaminomethyl)-3-hydroxy-3-phenyl-pentamethylen)-1,3,4,9-tetrahydro-pyrano[3,4-b]benzothiophen-citrat
1,1-(2-(Dimethylaminomethyl)-3-hydroxy-3-(3-fluorphenyl)-pentamethylen)-1,3,4,9-tetrahydro-pyrano[3,4-b]benzofuran-citrat
1, 1-(2-(Dimethylaminomethyl)-3-hydroxy-3-phenyl-pentamethylen)-1,3,4,9-tetrahydro-2-thia-9-aza-fluoren-citrat
1,1-(2-(Dimethylaminomethyl)-3-hydroxy-3-(3-fluorphenyl)-pentamethylen)-1,3,4,9-tetrahydro-pyrano[3,4-b]benzothiophen-citrat
1,1-(2-(Dimethylaminomethyl)-3-hydroxy-3-(2-methylphenyl)-pentamethylen)-1,3,4,9-tetrahydro-pyrano[3,4-b]benzothiophen-citrat
1,1-(2-(Dimethylaminomethyl)-3-hydroxy-3-(2-methylphenyl)-pentamethylen)-1,3,4,9-tetrahydro-pyrano[3,4-b]benzofuran-citrat
1,1-(2-(Dimethylaminomethyl)-3-hydroxy-3-(4-trifluormethylphenyl)-pentamethylen)-1,3,4,9-tetrahydro-pyrano[3,4-b]benzofuran-citrat
1,1-(2-(Dimethylaminomethyl)-3-hydroxy-3-(3-fluorphenyl)-pentamethylen)-1,3,4,9-tetrahydro-2-thia-9-aza-fluoren-citrat
1,1-(2-(Dimethylaminomethyl)-3-hydroxy-3-(4-trifluormethylphenyl)-pentamethylen)-3,4-dihydro-1*H*-2,9-diazafluoren dicitrat
1,1-(2-(Dimethylaminomethyl)-3-hydroxy-3-(3-fluorphenyl)-pentamethylen)-7-fluor-3,4-dihydro-1*H*-2,9-diazafluoren-sesquicitrat
1,1-(2-(Dimethylaminomethyl)-3-hydroxy-3-phenyl-pentamethylen)-3,4-dihydro-6-methoxy-1*H*-2,9-diazafluoren citrat
1,1-(2-(Dimethylaminomethyl)-3-hydroxy-3-(4-methylthiophenyl)-pentamethylen)-1,3,4,9-tetrahydro-6-methoxy-pyrano[3,4-b]indol-citrat
1,1-(2-(Dimethylaminomethyl)-3-hydroxy-3-(2-methy-3-(2-methylphenyl)-pentamethylen)-1,3,4,9-tetrahydro-6-methoxy-pyrano[3,4-b]indol-citrat
1,1-(2-(Dimethylaminomethyl)-3-hydroxy-3-(4-methylthiophenyl)-pentamethylen)-1,3,4,9-tetrahydro-pyrano[3,4-b]indol-citrat
1,1-(2-(Dimethylaminomethyl)-3-hydroxy-3-(3-fluorphenyl)-pentamethylen)-3,4-dihydro-6-methoxy-1*H*-2,9-diazafluoren citrat
1,1-(2-(Dimethylaminomethyl)-3-hydroxy-3-(4-trifluormethylphenyl)-pentamethylen)-3,4-dihydro-6-methoxy-1*H*-2,9-diazafluoren citrat
1,1-(2-(Dimethylaminomethyl)-3-hydroxy-3-(4-methylthiophenyl)-pentamethylen)-1,3,4,9-tetrahydro-2-thia-9-aza-fluoren-sesquicitrat
1,1-(2-(Dimethylaminomethyl)-3-hydroxy-3-(2-methylphenyl)-pentamethylen)-1,3,4,9-tetrahydro-2-thia-9-aza-fluoren-citrat
1,1-(2-(Dimethylaminomethyl)-3-hydroxy-3-(2-methylphenyl)-pentamethylen)-1,3,4,9-tetrahydro-pyrano[3,4-b]indol-citrat
1,1-(2-(Dimethylaminomethyl)-3-hydroxy-3-(4-methylthiophenyl)-pentamethylen)-3,4-dihydro-1*H*-2,9-diazafluoren citrat
1,1-(2-(Dimethylaminomethyl)-3-hydroxy-3-(3-fluorphenyl)-pentamethylen)-1,3,4,9-tetrahydro-pyrano[3,4-b]indol-hydrochlorid
1,1-(2-(Dimethylaminomethyl)-3-hydroxy-3-phenyl-pentamethylen)-3,4-dihydro-1H-2,9-diazafluoren citrat
1,1 -(2-(Dimethylaminomethyl)-3-hydroxy-3-(4-trifluormethylphenyl)-pentamethylen)-1,3,4,9-tetrahydro-pyrano[3,4-b]benzothiophen-citrat
1,1-(2-(Dimethylaminomethyl)-3-hydroxy-3-(4-methylthiophenyl)-pentamethylen)-1,3,4,9-tetrahydro-pyrano[3,4-b]benzofuran-citrat
1,1-(2-(Dimethylaminomethyl)-3-hydroxy-3-(4-methylthiophenyl)-pentamethylen)-1,3,4,9-tetrahydro-pyrano[3,4-b]benzothiophen-citrat
1,1-(2-(Dimethylaminomethyl)-3-hydroxy-3-(2-methylphenyl)-pentamethylen)-7-fluor-3,4-dihydro-1*H*-2,9-diazafluoren citrat
1,1-(2-(Dimethylaminomethyl)-3-hydroxy-3-(4-trifluormethlphenyl)-pentamethylen)-7-fluor-3,4-dihydro-1*H*-2,9-diazafluoren citrat
1,1-(2-(Dimethylaminomethyl)-3-hydroxy-3-(4-methylthiophenyl)-pentamethylen)-7-fluor-3,4-dihydro-1*H*-2,9-diazafluoren citrat
1,1-(2-(Dimethylaminomethyl)-3-hydroxy-3-phenyl-pentamethylen)-7-fluor-3,4-dihydro-1*H*-2,9-diazafluoren dicitrat
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

12. Verfahren zur Herstellung von spirocyclischen Cyclohexanderivaten gemäß Anspruch 1, **dadurch gekennzeichnet, dass** ein Edukt der allgemeinen Formel II 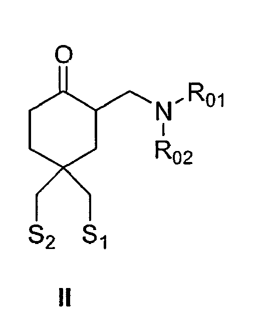 wobei die Reste R⁰¹ und R⁰² die für R1 und R² angegebene Bedeutung haben und zusätzlich für eine Schutzgruppe stehen können, in einem organischen Lösungsmittel mit einer metallorganischen Verbindung, vorzugsweise einer Grignard-Verbindung der allgemeinen Formel R³MgX oder einer Lithium-organischen Verbindungen R³-Li, umgesetzt wird und nach Abspaltung der Ketalschutzgruppe mit einer Verbindung der allgemeinen Formel B, wobei X O oder S bedeutet, 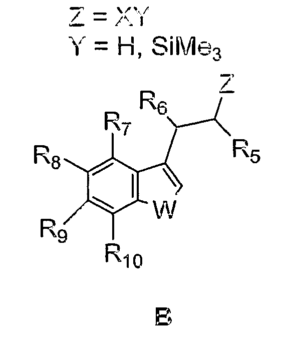 in Gegenwart geeigneter Kupplungsreagentien, beispielsweise starker Säuren oder deren Silylester umgesetzt wird.

13. Verfahren zur Herstellung von spirocyclischen Cyclohexanderivaten gemäß Anspruch 1, bei denen X NR¹⁷ bedeutet, **dadurch gekennzeichnet, dass** eine Verbindung der allgemeinen Formel A 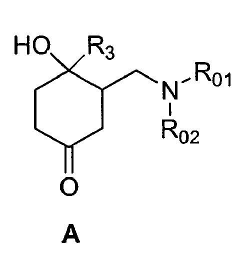 mit einer Verbindung der allgemeinen Formel Bₐ 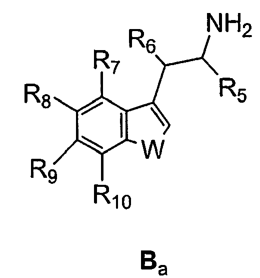 in Gegenwart geeigneter Kupplungsreagentien, beispielsweise starker Säuren oder deren Silylester umgesetzt wird.

14. Verfahren zur Herstellung von spirocyclischen Cyclohexanderivaten gemäß Anspruch 1, bei denen X NR⁷ und R¹⁷ COR¹² oder SO₂R¹² bedeutet, **dadurch gekennzeichnet, dass** ein spirocyclisches Cydohexanderivat, bei dem X NH bedeutet, unter Zugabe von Base, beispielsweise Triethylamin, mit einem Anhydrid oder einem Säurechlorid umgesetzt wird, vorzugsweise unter Mikrowelleneinstrahlung.

15. Verfahren zur Herstellung von spirocyclischen Cyclohexanderivaten gemäß Anspruch 1, bei denen X SO oder SO₂ bedeutet, **dadurch gekennzeichnet, dass** ein spirocyclisches Cyclohexanderivat, bei dem S bedeutet, mit Hilfe eines Oxidationsmittels, beispielsweise H₂O₂, oxidiert wird.

16. Arzneimittel enthaltend wenigstens ein spirocyclisches Cyclohexan -Derivat gemäß Anspruch 1, gegebenenfalls in Form seines Razemats, der reinen Stereoisomeren, insbesondere Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate, sowie gegebenenfalls enthaltend geeignete Zusatz- und/oder Hilfsstoffe und/oder gegebenenfalls weiterer Wirkstoffe.

17. Verwendung eines spirocyclischen Cyclohexan-Derivates gemäß Anspruch 1, gegebenenfalls in Form seines Razemats, der reinen Stereoisomeren, insbesondere Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate; zur Herstellung eines Arzneimittels zur Behandlung von Schmerz, insbesondere von akutem, neuropathischem oder chronischem Schmerz.

18. Verwendung eines spirocyclisches Cyclohexan-Derivats gemäß Anspruch 1 zur Herstellung eines Arzneimittels zur Behandlung von zur Behandlung von Angstzuständen, Depressionen, Epilepsie, cardiovaskulären Erkrankungen, Harninkontinenz, Diarrhöe, Pruritus, Alkohol- und Drogenmißbrauch, Medikamentenabhängigkeit, Entzündungen, Antriebslosigkeit, Bulimie, Anorexie, Katalepsie, zur Verwendung als Lokalanästhetikum, Antiarrythmikum, Antiemetikum, Nootropikum, und zur Vigilanz- und Libidosteigerung

## Claims

1. Spirocyclic cyclohexane derivatives of the general formula I 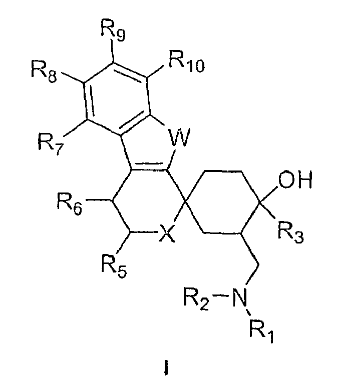 wherein
R¹ and R² independently of one another represent H; CHO; C₁₋₅-alkyl, in each case saturated or unsaturated, branched or unbranched, mono- or poly-substituted or unsubstituted; or aryl, C₃₋₈-cycloalkyl or heteroaryl bonded via C₁₋₃-alkyl, in each case mono- or poly-substituted or unsubstituted;
or the radicals R¹ and R² together represent CH₂CH₂OCH₂CH₂, CH₂CH₂NR¹¹CH₂CH₂ or (CH₂) ₃₋₆,
wherein R¹¹ represents H; C₁₋₅-alkyl, in each case saturated or unsaturated, branched or unbranched, mono- or poly-substituted or unsubstituted; C₃₋₈-cycloalkyl, in each case saturated or unsaturated, mono- or poly-substituted or unsubstituted; aryl or heteroaryl, in each case mono- or poly-substituted or unsubstituted; or aryl, C₃₋₈-cycloalkyl or heteroaryl bonded via C₁₋₃-alkyl, in each case mono-or poly-substituted or unsubstituted;
R³ represents phenyl, phenethyl, thiophenyl, pyridyl or benzyl, in each case unsubstituted or mono- or poly-substituted;
W represents NR⁴, O or S
and
R⁴ represents H; C₁₋₅-alkyl, saturated or unsaturated, branched or unbranched, unsubstituted or mono- or poly-substituted; aryl or heteroaryl, in each case substituted or unsubstituted; aryl, heteroaryl or cycloalkyl bonded via a C₁₋₃-alkyl group, in each case mono- or poly-substituted or unsubstituted; COR¹² ; SO₂R¹²;
wherein R¹² represents H; C₁₋₅-alkyl, in each case saturated or unsaturated, branched or unbranched, mono- or poly-substituted or unsubstituted; C₃₋₈-cycloalkyl, in each case saturated or unsaturated, mono- or poly-substituted or unsubstituted; aryl or heteroaryl, in each case mono- or poly-substituted or unsubstituted; or aryl, C₃-₈-cycloalkyl or heteroaryl bonded via C₁₋₃-alkyl, in each case mono- or poly-substituted or unsubstituted; OR¹³; NR¹⁴R¹⁵;
R⁵ represents =O; H; COOR¹³, CONR¹³, OR¹³; C₁₋₅-alkyl, saturated or unsaturated, branched or unbranched, unsubstituted or mono- or poly-substituted; C₃₋₈-cycloalkyl, saturated or unsaturated, unsubstituted or mono- or poly-substituted; aryl or heteroaryl, unsubstituted or mono- or poly-substituted; or aryl, C₃₋₈-cycloalkyl or heteroaryl bonded via C₁₋₃-alkyl, unsubstituted or mono- or poly-substituted;
R⁶ represents H; F, Cl, NO₂, CF₃, OR¹³, SR¹³, SO₂R¹³, SO₂OR¹³, CN, COOR¹³, NR¹⁴R¹⁵; C₁₋₅-alkyl, saturated or unsaturated, branched or unbranched, unsubstituted or mono- or poly-substituted; C₃₋₈-cycloalkyl, saturated or unsaturated, unsubstituted or mono- or poly-substituted; aryl or heteroaryl, unsubstituted or mono- or poly-substituted; or aryl, C₃₋₈-cycloalkyl or heteroaryl bonded via C₁₋₃-alkyl, unsubstituted or mono- or poly-substituted;
or R⁵ and R⁶ together represent (CH₂)ₙ wherein n = 2, 3, 4, 5 or 6, wherein individual hydrogen atoms can also be replaced by F, Cl, Br, I, NO₂, CF₃, OR¹³, CN or C₁₋₅-alkyl;
R⁷, R⁸, R⁹ and R¹⁰ independently of one another represent H, F, Cl, Br, I, NO₂, CF₃, OR¹³, SR¹³, SO₂R¹³, SO₂OR¹³, CN, COOR¹³, NR¹⁴R¹⁵ ; C₁₋₅-alkyl, C₃₋₈-cycloalkyl, unsubstituted or mono- or poly-substituted; aryl or heteroaryl, unsubstituted or mono- or poly-substituted; or aryl, C₃₋₈-cycloalkyl or heteroaryl bonded via C₁₋₃-alkyl, unsubstituted or mono- or poly-substituted;
wherein R¹³ represents H; C₁₋₅-alkyl, in each case saturated or unsaturated, branched or unbranched, unsubstituted or mono- or poly-substituted; C₃₋₈-cycloalkyl, in each case saturated or unsaturated, unsubstituted or mono- or poly-substituted; aryl or heteroaryl, unsubstituted or mono- or poly-substituted; or aryl, C₃₋₈-cycloalkyl or heteroaryl bonded via C₁₋₃-alkyl, unsubstituted or mono- or poly-substituted;
R¹⁴ and R¹⁵ independently of one another represent H; C₁₋₅-alkyl, in each case saturated or unsaturated, branched or unbranched, unsubstituted or mono- or poly-substituted; or C₃₋₈-cycloalkyl, in each case saturated or unsaturated, unsubstituted or mono- or poly-substituted; aryl or heteroaryl, unsubstituted or mono- or poly-substituted; or aryl, C₃₋₈-cycloalkyl or heteroaryl bonded via C₁₋₃-alkyl, unsubstituted or mono- or poly-substituted;
or R¹⁴ and R¹⁵ together form CH₂CH₂OCH₂CH₂, CH₂CH₂NR¹⁶CH₂CH₂ or (CH₂) ₃₋₆,
wherein R¹⁶ represents H; C₁₋₅ -alkyl, saturated or unsaturated, branched or unbranched, unsubstituted or mono- or poly-substituted;
X represents O, S, SO, SO₂ or NR¹⁷;
R¹⁷ represents H; C₁₋₅-alkyl, saturated or unsaturated, branched or unbranched; COR¹² or SO₂R¹²; in the form of the racemate; of the enantiomers, diastereoisomers, mixtures of the enantiomers or diastereoisomers or in the form of an individual enantiomer or diastereoisomer; in the form of the bases and/or salts of physiologically acceptable acids or cations.

2. Spirocyclic cyclohexane derivatives according to claim 1, **characterised in that**
R¹ and R² independently of one another represent H; C₁₋₅-alkyl, saturated or unsaturated, branched or unbranched, mono- or poly-substituted or unsubstituted;
or the radicals R¹ and R² together form a ring and represent CH₂CH₂OCH₂CH₂, CH₂CH₂NR¹¹CH₂CH₂ or (CH₂)₃₋₆,
wherein R¹¹ represents H; C₁₋₅-alkyl, saturated or unsaturated, branched or unbranched, mono- or poly-substituted or unsubstituted.

3. Spirocyclic cyclohexane derivatives according to claim 1, **characterised in that** R¹ and R² independently of one another represent CH₃ or H, wherein R¹ and R² do not simultaneously represent H.

4. Spirocyclic cyclohexane derivatives according to any one of claims 1 to 3, **characterised in that** R³ represents phenyl, unsubstituted or mono- or poly-substituted.

5. Spirocyclic cyclohexane derivatives according to any one of claims 1 to 4, **characterised in that** R³ represents phenyl, unsubstituted or mono- or poly-substituted by F, Cl, CN, OCH₃, SCH₃, OCH₂CH₃, CH₃, CF₃ or by OH, in particular phenyl, 3-methoxyphenyl, 2-methylphenyl, 4-trifluoromethylphenyl, 4-methylthiophenyl, 3-fluorophenyl and 4-fluorophenyl.

6. Spirocyclic cyclohexane derivatives according to any one of claims 1 to 5, **characterised in that** W represents NR⁴, O or S and X represents NR¹⁷, O or S.

7. Spirocyclic cyclohexane derivatives according to any one of claims 1 to 5, **characterised in that** W represents NH, O or S and X represents NH, NC(O)CH₃, O or S.

8. Spirocyclic cyclohexane derivatives according to any one of claims 1 to 7, **characterised in that** R⁷, R⁸, R⁹ and R¹⁰ independently of one another represent H; C₁₋₅-alkyl, branched or unbranched, unsubstituted or mono-or poly-substituted; F, Cl, Br, I, CF₃, OH, OCH₃, NH₂, COOH, COOCH₃ , NHCH₃ or N(CH₃)₂ or NO₂, in particular H, F or OCH₃.

9. Spirocyclic cyclohexane derivatives according to any one of claims 1 to 8, **characterised in that** R⁵ represents H, C₁₋₅-alkyl, branched or unbranched, unsubstituted or mono- or poly-substituted, or COOR¹³ and R⁶ represents H or C₁₋₅-alkyl, in particular R⁵ and R⁶ represent H.

10. Spirocyclic cyclohexane derivatives according to any one of claims 1 to 7, **characterised in that** the radicals R⁵ to R¹⁰ represent H.

11. Spirocyclic cyclohexane derivatives according to any one of claims 1 to 10 from the group
1,1-(2-(dimethylaminomethyl)-3-hydroxy-3-(3-methoxyphenyl)pentamethylene)-3,4-dihydro-1H-2,9-diazafluorene dihydrochloride
1,1-(2-(dimethylaminomethyl)-3-hydroxy-3-(3-methoxyphenyl)pentamethylene)-2-acetyl-3,4-dihydro-1H-2,9-diazafluorene hydrochloride
1,1-(2-(dimethylaminomethyl)-3-hydroxy-3-(3-fluorophenyl)pentamethylene)-3,4-dihydro-1*H*-2,9-diazafluorene citrate
1,1-(2-(dimethylaminomethyl)-3-hydroxy-3-phenyl-pentamethylene)-1,3,4,9-tetrahydro-pyrano[3,4-b]indole sesquicitrate
1,1-(2-(dimethylaminomethyl)-3-hydroxy-3-phenyl-pentamethylene)-1,3,4,9-tetrahydro-pyrano[3,4-b]-benzofuran citrate
1,1-(2-(dimethylaminomethyl)-3-hydroxy-3-(2-methylphenyl)-pentamethylene)-3,4-dihydro-1*H*-2,9-diazafluorene citrate
1,1-(2-(dimethylaminomethyl)-3-hydroxy-3-phenyl-pentamethylene)-1,3,4,9-tetrahydro-pyrano[3,4-b]-benzothiophene citrate
1,1-(2-(dimethylaminomethyl)-3-hydroxy-3-(3-fluorophenyl)-pentamethylene)-1,3,4,9-tetrahydro-pyrano-[3,4-b]benzofuran citrate
1,1-(2-(dimethylaminomethyl)-3-hydroxy-3-phenyl-pentamethylene)-1,3,4,9-tetrahydro-2-thia-9-aza-fluorene citrate
1,1-(2-(dimethylaminomethyl)-3-hydroxy-3-(3-fluorophenyl)-pentamethylene)-1,3,4,9-tetrahydro-pyrano-[3,4-b]benzothiophene citrate
1,1-(2-(dimethylaminomethyl)-3-hydroxy-3-(2-methylphenyl)-pentamethylene)-1,3,4,9-tetrahydro-pyrano-[3,4-b]benzothiophene citrate
1,1-(2-(dimethylaminomethyl)-3-hydroxy-3-(2-methylphenyl)-pentamethylene)-1,3,4,9-tetrahydro-pyrano-[3,4-b]benzofuran citrate
1,1-(2-(dimethylaminomethyl)-3-hydroxy-3-(4-trifluoromethylphenyl)-pentamethylene)-1,3,4,9-tetrahydropyrano[3,4-b]benzofuran citrate
1,1-(2-(dimethylaminomethyl)-3-hydroxy-3-(3-fluorophenyl)-pentamethylene)-1,3,4,9-tetrahydro-2-thia-9-aza-fluorene citrate
1,1-(2-(dimethylaminomethyl)-3-hydroxy-3-(4-trifluoromethylphenyl)-pentamethylene)-3,4-dihydro-1*H*-2,9-diazafluorene dicitrate
1,1-(2-(dimethylaminomethyl)-3-hydroxy-3-(3-fluorophenyl)-pentamethylene)-7-fluoro-3,4-dihydro-1*H*-2,9-diazafluorene sesquicitrate
1,1-(2-(dimethylaminomethyl)-3-hydroxy-3-phenyl-pentamethylene)-3,4-dihydro-6-methoxy-1*H*-2,9-diazafluorene citrate
1,1-(2-(dimethylaminomethyl)-3-hydroxy-3-(4-methylthiophenyl)-pentamethylene)-1,3,4,9-tetrahydro-6-methoxy-pyrano[3,4-b]indole citrate
1,1-(2-(dimethylaminomethyl)-3-hydroxy-3-(2-methylphenyl)-pentamethylene)-1,3,4,9-tetrahydro-6-methoxy-pyrano[3,4-b]indole citrate
1,1-(2-(dimethylaminomethyl)-3-hydroxy-3-(4-methylthiophenyl)-pentamethylene)-1,3,4,9-tetrahydropyrano [3, 4-b] indole citrate
1,1-(2-(dimethylaminomethyl)-3-hydroxy-3-(3-fluorophenyl)-pentamethylene)-3,4-dihydro-6-methoxy-1H-2,9-diazafluorene citrate
1,1-(2-(dimethylaminomethyl)-3-hydroxy-3-(4-trifluoromethylphenyl)-pentamethylene)-3,4-dihydro-6-methoxy-1*H*-2,9-diazafluorene citrate
1,1-(2-(dimethylaminomethyl)-3-hydroxy-3-(4-methylthiophenyl)-pentamethylene)-1,3,4,9-tetrahydro-2-thia-9-aza-fluorene sesquicitrate
1,1-(2-(dimethylaminomethyl)-3-hydroxy-3-(2-methylphenyl)-pentamethylene)-1,3,4,9-tetrahydro-2-thia-9-aza-fluorene citrate
1,1-(2-(dimethylaminomethyl)-3-hydroxy-3-(2-methylphenyl)-pentamethylene)-1,3,4,9-tetrahydro-pyrano-[3,4-b] indole citrate
1,1-(2-(dimethylaminomethyl)-3-hydroxy-3-(4-methylthiophenyl)-pentamethylene)-3,4-dihydro-1*H*-2,9-diazafluorene citrate
1,1-(2-(dimethylaminomethyl)-3-hydroxy-3-(3-fluorophenyl)-pentamethylene)-1,3,4,9-tetrahydro-pyrano-[3,4-b]indole hydrochloride
1,1-(2-(dimethylaminomethyl)-3-hydroxy-3-phenyl-pentamethylene)-3,4-dihydro-1H-2,9-diazafluorene citrate
1,1-(2-(dimethylaminomethyl)-3-hydroxy-3-(4-trifluoromethylphenyl)-pentamethylene)-1,3,4,9-tetrahydropyrano[3,4-b]benzothiophene citrate
1,1-(2-(dimethylaminomethyl)-3-hydroxy-3-(4-methylthiophenyl)-pentamethylene)-1,3,4,9-tetrahydropyrano[3,4-b]benzofuran citrate
1,1-(2-(dimethylaminomethyl)-3-hydroxy-3-(4-methylthiophenyl)-pentamethylene)-1,3,4,9-tetrahydropyrano[3,4-b]benzothiophene citrate
1,1-(2-(dimethylaminomethyl)-3-hydroxy-3-(2-methylphenyl)-pentamethylene)-7-fluoro-3,4-dihydro-1*H*-2,9-diazafluorene citrate
1,1-(2-(dimethylaminomethyl)-3-hydroxy-3-(4-trifluoromethylphenyl)-pentamethylene)-7-fluoro-3,4-dihydro-1*H*-2,9-diazafluorene citrate
1,1-(2-(dimethylaminomethyl)-3-hydroxy-3-(4-methylthiophenyl)-pentamethylene)-7-fluoro-3,4-dihydro-1*H-*2,9-diazafluorene citrate
1,1-(2-(dimethylaminomethyl)-3-hydroxy-3-phenyl-pentamethylene)-7-fluoro-3,4-dihydro-1*H*-2,9-diazafluorene dicitrate
in the form of the racemate; of the enantiomers, diastereoisomers, mixtures of the enantiomers or diastereoisomers or in the form of an individual enantiomer or diastereoisomer; in the form of the bases and/or salts of physiologically acceptable acids or cations.

12. Process for the preparation of spirocyclic cyclohexane derivatives according to claim 1, **characterised in that** a starting material of the general formula II 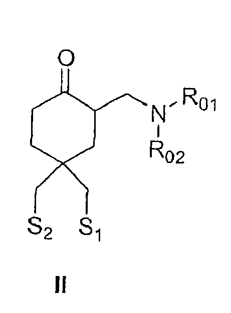 wherein the radicals R⁰¹ and R⁰² are as defined for R¹ and R² and can additionally represent a protecting group, is reacted in an organic solvent with an organometallic compound, preferably a Grignard compound of the general formula R³MgX or an organolithium compound R³-Li, and, after removal of the ketal protecting group, is reacted with a compound of the general formula B, wherein X represents O or S, 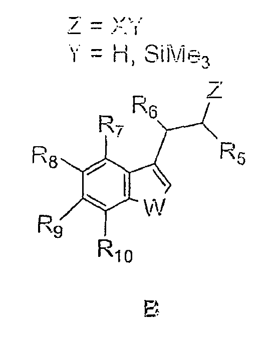 in the presence of suitable coupling reagents, for example strong acids or silyl esters thereof.

13. Process for the preparation of spirocyclic cyclohexane derivatives according to claim 1 wherein X represents NR¹⁷, **characterised in that** a compound of the general formula A 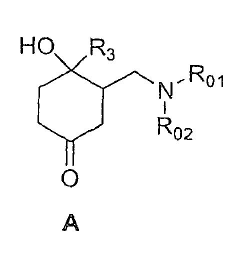 is reacted with a compound of the general formula Bₐ 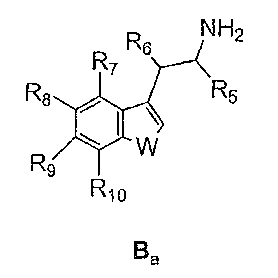 in the presence of suitable coupling reagents, for example strong acids or silyl esters thereof.

14. Process for the preparation of spirocyclic cyclohexane derivatives according to claim 1 wherein X represents NR¹⁷ and R¹⁷ represents COR¹² or SO₂R¹², **characterised in that** a spirocyclic cyclohexane derivative wherein X represents NH is reacted, with the addition of a base, for example triethylamine, with an anhydride or an acid chloride, preferably with microwave irradiation.

15. Process for the preparation of spirocyclic cyclohexane derivatives according to claim 1 wherein X represents SO or SO₂, **characterised in that** a spirocyclic cyclohexane derivative wherein X represents S is oxidised by means of an oxidising agent, for example H₂O₂.

16. Medicament comprising at least one spirocyclic cyclohexane derivative according to claim 1, optionally in the form of its racemate, of the pure stereoisomers, in particular enantiomers and diastereoisomers, in any desired mixing ratio; in the form of its acids or its bases or in the form of its salts, in particular the physiologically acceptable salts or salts of physiologically acceptable acids or cations; or in the form of its solvates, in particular the hydrates, and optionally comprising suitable additives and/or auxiliary substances and/or optionally further active ingredients.

17. Use of a spirocyclic cyclohexane derivative according to claim 1, optionally in the form of its racemate, of the pure stereoisomers, in particular enantiomers and diastereoisomers, in any desired mixing ratio; in the form of its acids or its bases or in the form of its salts, in particular the physiologically acceptable salts or salts of physiologically acceptable acids or cations; or in the form of its solvates, in particular the hydrates; in the preparation of a medicament for the treatment of pain, in particular of acute, neuropathic or chronic pain.

18. Use of a spirocyclic cyclohexane derivative according to claim 1 in the preparation of a medicament for the treatment of anxiety, depression, epilepsy, cardiovascular diseases, urinary incontinence, diarrhoea, pruritus, alcohol and drug abuse, medicament dependency, inflammations, lack of drive, bulimia, anorexia, catalepsy, for use as a local anaesthetic, anti-arrhythmic, anti-emetic, nootropic agent, and for increasing vigilance and libido.

## Revendications

1. Dérivés spirocycliques de cyclohexane de formule générale I 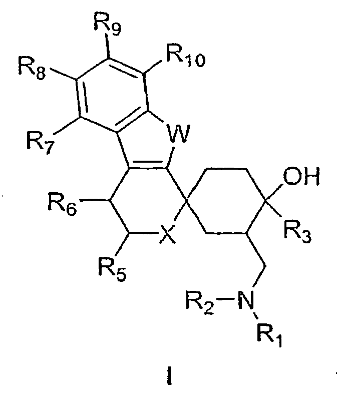 dans laquelle
R¹ et R² représentent, indépendamment l'un de l'autre, H ; un groupe CHO ; un reste alkyle en C₁ à C₅, dans chaque cas saturé ou non saturé, ramifié ou non ramifié, substitué une ou plusieurs fois ou non substitué ; ou un reste hétéroaryle, cycloalkyle en C₃ à C₈ ou aryle lié par l'intermédiaire d'un radical alkyle en C₁ à C₃, chacun substitué une ou plusieurs fois ou non substitué ;
ou bien les restes R¹ et R² forment ensemble un groupe CH₂CH₂OCH₂CH₂, CH₂CH₂NR¹¹CH₂CH₂ ou (CH₂) ₃₋₆,
R¹¹ représentant H ; un reste alkyle en C₁ à C₅, dans chaque cas saturé ou non saturé, ramifié ou non ramifié, substitué une ou plusieurs fois ou non substitué ; un reste cycloalkyle en C₃ à C₈, dans chaque cas saturé ou non saturé, substitué une ou plusieurs fois ou non substitué ; un reste aryle ou hétéroaryle, dans chaque cas substitué ou une plusieurs fois ou non substitué ; ou un reste hétéroaryle, cycloalkyle en C₃ à C₈ ou aryle lié par l'intermédiaire d'un radical alkyle en C₁ à C₃, chacun substitué une ou plusieurs fois ou non substitué ;
R³ représente un reste phényle, phénéthyle, thiophényle, pyridyle ou benzyle, chacun non substitué ou substitué une ou plusieurs fois ;
W représente NR⁴, O ou S et
R⁴ représente H ; un reste alkyle en C₁ à C₅, saturé ou non saturé, ramifié ou non ramifié, non substitué ou substitué une ou plusieurs fois ; un reste aryle ou hétéroaryle, dans chaque cas substitué ou non substitué ou un reste hétéroaryle, cycloalkyle en C₃ à C₈ ou aryle lié par l'intermédiaire d'un radical alkyle en C₁ à C₃, chacun substitué une ou plusieurs fois ou non substitué ;
un groupe COR¹² ; SO₂R¹²,
R¹² représentant H, un reste alkyle en C₁ à C₅, dans chaque cas saturé ou non saturé, ramifié ou non ramifié, substitué une ou plusieurs fois ou non substitué ; un reste cycloalkyle en C₃ à C₈, dans chaque cas saturé ou non saturé, substitué une ou plusieurs fois ou non substitué ; un reste aryle ou hétéroaryle, dans chaque cas substitué une ou plusieurs fois ou non substitué ; ou un reste hétéroaryle, cycloalkyle en C₃ à C₈ ou aryle lié par l'intermédiaire d'un radical alkyle en C₁ à C₃, chacun substitué une ou plusieurs fois ou non substitué ; un groupe OR¹³ ; NR¹⁴R¹⁵ ;
R⁵ représente =O ; H ; un groupe COOR¹³ , CONR¹³ , OR¹³ ; un reste alkyle en C₁ à C₅, saturé ou non saturé, ramifié ou non ramifié, non substitué ou substitué une ou plusieurs fois ; un reste cycloalkyle en C₃ à C₈, saturé ou non saturé, non substitué ou substitué une ou plusieurs fois ; un reste aryle ou hétéroaryle, non substitué ou substitué une ou plusieurs fois ; ou un reste hétéroaryle, cycloalkyle en C₃ à C₈ ou aryle lié par l'intermédiaire d'un radical alkyle en C₁ à C₃, chacun substitué une ou plusieurs fois ou non substitué ;
R⁶ représente H ; F, Cl, NO₂, CF₃, OR¹³, SR¹³, SO₂R¹³, SO₂OR¹³, CN, COOR¹³, NR¹⁴R¹⁵, un reste alkyle en C₁ à C₅, saturé ou non saturé, ramifié ou non ramifié, non substitué ou substitué une ou plusieurs fois ; un reste cycloalkyle en C₃ à C₈, saturé ou non saturé, non substitué ou substitué une ou plusieurs fois ; un reste aryle ou hétéroaryle, non substitué ou substitué une ou plusieurs fois ; ou un reste hétéroaryle, cycloalkyle en C₃ à C₈ ou aryle lié par l'intermédiaire d'un radical alkyle en C₁ à C₃, chacun substitué une ou plusieurs fois ou non substitué ;
ou bien R⁵ et R⁶ forment ensemble un groupe (CH₂) ₙ dans lequel n a la valeur 2, 3, 4, 5 ou 6, des atomes d'hydrogène individuels pouvant aussi être remplacés par F, Cl, Br, I, NO₂, CF₃, OR¹³, un groupe CN ou alkyle en C₁ à C₅ ;
R⁷, R⁸, R⁹ et R¹⁰ représentent, indépendamment les uns des autres, H, F, Cl, Br, I, NO₂, CF₃, OR¹³, SR¹³, SO₂R¹³, SO₂OR¹³, CN, COOR¹³, NR¹⁴R¹⁵, un reste alkyle en C₁ à C₅, cycloalkyle en C₃ à C₈, non substitué ou substitué une ou plusieurs fois ; un reste aryle ou hétéroaryle, non substitué ou substitué une ou plusieurs fois ; ou un reste hétéroaryle, cycloalkyle en C₃ à C₈ ou aryle lié par l'intermédiaire d'un radical alkyle en C₁ à C₃, chacun substitué une ou plusieurs fois ou non substitué ;
R¹³ représentant H, un radical alkyle en C₁ à C₅, dans chaque cas saturé ou non saturé, ramifié ou non ramifié, non substitué ou substitué une ou plusieurs fois ; un radical cycloalkyle en C₃ à C₈, dans chaque cas saturé ou non saturé, non substitué ou substitué une ou plusieurs fois ; un radical aryle ou hétéroaryle, non substitué ou substitué une ou plusieurs fois ; ou un reste hétéroaryle, cycloalkyle en C₃ à C₈ ou aryle lié par l'intermédiaire d'un radical alkyle en C₁ à C₃, chacun substitué une ou plusieurs fois ou non substitué ;
R¹⁴ et R¹⁵ représentent indépendamment l'un de l'autre, H, un reste alkyle en C₁ à C₅, dans chaque cas saturé ou non saturé, ramifié ou non ramifié, non substitué ou substitué une ou plusieurs fois ; ou un radical cycloalkyle en C₃ à C₈, dans chaque cas saturé ou non saturé, non substitué ou substitué une ou plusieurs fois ; un reste aryle ou hétéroaryle, non substitué ou substitué une ou plusieurs fois ; ou un reste hétéroaryle, cycloalkyle en C₃ à C₈ ou aryle lié par l'intermédiaire d'un radical alkyle en C₁ à C₃, chacun substitué une ou plusieurs fois ou non substitué ;
ou bien R¹⁴ et R¹⁵ forment ensemble un groupe CH₂CH₂OCH₂CH₂, CH₂CH₂NR¹⁶CH₂CH₂ ou (CH₂)₃₋₆,
R¹⁶ représentant H ; un radical alkyle en C₁ à C₅ saturé ou non saturé, ramifié ou non ramifié, non substitué ou substitué une ou plusieurs fois ;
X représente O, S, SO, SO₂ ou NR¹⁷;
R¹⁷ représente H ; un radical alkyle en C₁ à C₅, saturé ou non saturé, ramifié ou non ramifié ; un groupe COR¹² ou SO₂R¹² ,
sous forme du racémate ; des énantiomères, des diastéréoisomères, de mélanges des énantiomères ou des diastéréoisomères ou sous forme d'un énantiomère ou d'un diastéréoisomère individuel ; des bases et/ou des sels d'acides ou de cations physiologiquement acceptables.

2. Dérivés spirocycliques de cyclohexane suivant la revendication 1, **caractérisés en ce que**
R¹ et R² représentent, indépendamment l'un de l'autre, H ; un reste alkyle en C₁ à C₅, saturé ou non saturé, ramifié ou non ramifié, substitué une ou plusieurs fois ou non substitué ;
ou bien les restes R¹ et R² forment ensemble un noyau et représentent CH₂CH₂OCH₂CH₂, CH₂CH₂NR¹¹CH₂CH₂ ou (CH₂) ₃₋₆.
R¹¹ représentant H ; un radical alkyle en C₁ à C₅, saturé ou non saturé, ramifié ou non ramifié, substitué une
ou plusieurs fois ou non substitué.

3. Dérivés spirocycliques de cyclohexane suivant la revendication 1, **caractérisés en ce que** R¹ et R² représentent, indépendamment l'un de l'autre, CH₃ ou H, R¹ et R² ne représentant pas en même temps H.

4. Dérivés spirocycliques de cyclohexane suivant l'une des revendications 1 à 3, **caractérisés en ce que** R³ est un reste phényle, non substitué ou substitué une ou plusieurs fois.

5. Dérivés spirocycliques de cyclohexane suivant l'une des revendications 1 à 4, **caractérisés en ce que** R³ représente un reste phényle, non substitué ou substitué une ou plusieurs fois avec F, Cl, CN, OCH₃, SCH₃, OCH₂CH₃, CH₃, CF₃ ou OH, en particulier des restes phényle, 3-méthoxyphényle, 2-méthylphényle, 4-trifluorométhylphényle, 4-méthylthiophényle, 3-fluorophényle et 4-fluorophényle.

6. Dérivés spirocycliques de cyclohexane suivant l'une des revendications 1 à 5, **caractérisés en ce que** W représente un groupe NR⁴, O ou S, et X représente un groupe NR¹⁷ O ou S.

7. Dérivés spirocycliques de cyclohexane suivant l'une des revendications 1 à 5, **caractérisés en ce que** W représente un groupe NH, O ou S, et X représente NH, NC (O) CH₃ , O ou S.

8. Dérivés spirocycliques de cyclohexane suivant l'une des revendications 1 à 7, **caractérisés en ce que** R⁷, R⁸, R⁹ et R¹⁰ représentent, indépendamment les uns des autres, H ; un groupe alkyle en C₁ à C₅, ramifié ou non ramifié, non substitué ou substitué une ou plusieurs fois ; F, Cl, Br, I, CF₃, OH, OCH₃ , NH₂, COOH, COOCH₃, NHCH₃ ou N(CH₃)₂ ou NO₂, en particulier H, F ou OCH₃.

9. Dérivés spirocycliques de cyclohexane suivant l'une des revendications 1 à 8, **caractérisés en ce que** R⁵ représente H, un reste alkyle en C₁ à C₅, ramifié ou non ramifié, non substitué ou substitué une ou plusieurs fois
ou un groupe COOR¹³, et R⁶ représente H ou un reste alkyle en C₁ à C₅, R⁵ et R⁶ représentant en particulier H.

10. Dérivés spirocycliques de cyclohexane suivant l'une des revendications 1 à 7, **caractérisés en ce que** les restes R⁵ à R¹⁰ représentent H.

11. Dérivés spirocycliques de cyclohexane suivant l'une des revendications 1 à 10, choisis dans le groupe :
dichlorhydrate de 1,1-(2-(diméthylaminométhyl)-3-hydroxy-3-(3-méthoxyphényl)pentaméthylène)-3,4-dihydro-1H-2,9-diazafluorène
chlorhydrate de 1,1-(2-(diméthylaminométhyl)-3-hydroxy-3-(3-méthoxyphényl)pentaméthylène)-2-acétyl-3,4-dihydro-1H-2,9-diazafluorène
citrate de 1,1-(2-(diméthylaminométhyl)-3-hydroxy-3-(3-fluorophényl)pentaméthylène)-3,4-dihydro-1H-2,9-diazafluorène
sesquicitrate de 1,1-(2-(diméthylaminométhyl)-3-hydroxy-3-phényl-pentaméthylène)-1,3,4,9-tétrahydro-pyranno-[3, 4-b] indole
citrate de 1,1-(2-(diméthylaminométhyl)-3-hydroxy-3-phényl-pentaméthylène)-1,3,4,9-tétrahydro-pyranno[3,4-b]-benzofuranne
citrate de 1,1-(2-(diméthylaminométhyl)-3-hydroxy-3-(2-méthylphényl)pentaméthylène)-3,4-dihydro-1H-2,9-diazafluorène
citrate de 1,1-(2-(diméthylaminométhyl)-3-hydroxy-3-phényl-pentaméthylène)-1,3,4,9-tétrahydro-pyranno[3,4-b]-benzothiophène
citrate de 1,1-(2-(diméthylaminométhyl)-3-hydroxy-3-(3-fluorophényl)pentaméthylène)-1,3,4,9-tétrahydropyranno[3,4-b]benzofuranne
citrate de 1,1-(2-(diméthylaminométhyl)-3-hydroxy-3-phényl-pentaméthylène)-1,3,4,9-tétrahydro-2-thia-9-azafluorène
citrate de 1,1-(2-(diméthylaminométhyl)-3-hydroxy-3-(3-fluorophényl)pentaméthylène)-1,3,4,9-tétrahydropyranno[3,4-b] benzothiophène
citrate de 1,1-(2-(diméthylaminométhyl)-3-hydroxy-3-(2-méthylphényl)pentaméthylène)-1,3,4,9-tétrahydropyranno [3,4-b] benzothiophène
citrate de 1,1-(2-(diméthylaminométhyl)-3-hydroxy-3-(2-méthylphényl)pentaméthylène)-1,3,4,9-tétrahydropyranno [3,4-b] benzofuranne
citrate de 1,1-(2-(diméthylaminométhyl)-3-hydroxy-3-(4-trifluorométhylphényl)pentaméthylène)-1,3,4,9-tétrahydro-pyranno[3,4-b]benzofuranne
citrate de 1,1-(2-(diméthylaminométhyl)-3-hydroxy-3-(3-fluorophényl)pentaméthylène)-1,3,4,9-tétrahydro-2-thia-9-azafluorène
dicitrate de 1,1-(2-(diméthylaminométhyl)-3-hydroxy-3-(4-trifluorométhylphényl)pentaméthylène)-3,4-dihydro-1*H-*2,9-diazafluorène
sesquicitrate de 1,1-(2-(diméthylaminométhyl)-3-hydroxy-3-(3-fluorophényl)pentaméthylène)-7-fluoro-3,4-dihydro-1*H*-2,9-diazafluorène
citrate de 1,1-(2-(diméthylaminométhyl)-3-hydroxy-3-phényl-pentaméthylène)-3,4-dihydro-6-méthoxy-1*H*-2,9-diazafluorène
citrate de 1,1-(2-(diméthylaminométhyl)-3-hydroxy-3-(4-méthylthiophényl)pentaméthylène)-1,3,4,9-tétrahydro-6-méthoxy-pyranno [3,4-b] indole
citrate de 1,1-(2-(diméthylaminométhyl)-3-hydroxy-3-(2-méthylphényl)pentaméthylène)-1,3,4,9-tétrahydro-6-méthoxy-pyranno [3,4-b]indole
citrate de 1,1-(2-(diméthylaminométhyl)-3-hydroxy-3-(4-méthylthiophényl)pentaméthylène)-1,3,4,9-tétrahydropyranno [3,4-b] indole
citrate de 1,1-(2-(diméthylaminométhyl)-3-hydroxy-3-(3-fluorophényl)pentaméthylène)-3,4-dihydro-6-méthoxy-1*H-*2,9-diazafluorène
citrate de 1,1-(2-(diméthylaminométhyl)-3-hydroxy-3-(4-trifluorométhylphényl)pentaméthylène)-3,4-dihydro-6-méthoxy-1*H*-2,9-diazafluorène
sesquicitrate de 1,1-(2-(diméthylaminométhyl)-3-hydroxy-3-(4-méthylthiophényl)pentaméthylène)-1,3,4,9-tétrahydro-2-thia-9-azafluorène
citrate de 1,1-(2-(diméthylaminométhyl)-3-hydroxy-3-(2-méthylphényl)pentaméthylène)-1,3,4,9-tétrahydro-2-thia-9-azafluorène
citrate de 1,1-(2-(diméthylaminométhyl)-3-hydroxy-3-(2-méthylphényl)pentaméthylène)-1,3,4,9-tétrahydropyranno [3,4-b] indole
citrate de 1,1-(2-(diméthylaminométhyl)-3-hydroxy-3-(4-méthylthiophényl)pentaméthylène)-3,4-dihydro-1*H*-2,9-diazafluorène
chlorhydrate de 1,1-(2-(diméthylaminométhyl)-3-hydroxy-3-(3-fluorophényl)pentaméthylène)-1,3,4,9-tétrahydro-pyranno[3,4-b]indole
citrate de 1,1-(2-(diméthylaminométhyl)-3-hydroxy-3-phényl-pentaméthylène)-3,4-dihydro-1H-2,9-diazafluorène
citrate de 1,1-(2-(diméthylaminométhyl)-3-hydroxy-3-(4-trifluorométhylphényl)pentaméthylène)-1,3,4,9-tétrahydro-pyranno[3,4-b]benzothiophène
citrate de 1,1-(2-(diméthylaminométhyl)-3-hydroxy-3-(4-méthylthiophényl)pentaméthylène)-1,3,4,9-tétrahydropyranno [3,4-b] benzofuranne
citrate de 1,1-(2-(diméthylaminométhyl)-3-hydroxy-3-(4-méthylthiophényl)pentaméthylène)-1,3,4,9-tétrahydropyranno [3,4-b] benzothiophène
citrate de 1,1-(2-(diméthylaminométhyl)-3-hydroxy-3-(2-méthylphényl)pentaméthylène)-7-fluoro-3,4-dihydro-1*H-*2,9-diazafluorène
citrate de 1,1-(2-(diméthylaminométhyl)-3-hydroxy-3-(4-trifluorométhylphényl)pentaméthylène)-7-fluoro-3,4-dihydro-1*H*-2,9-diazafluorène
citrate de 1,1-(2-(diméthylaminométhyl)-3-hydroxy-3-(4-méthylthiophényl)pentaméthylène)-7-fluoro-3,4-dihydro-1*H*-2,9-diazafluorène
dicitrate de 1,1-(2-(diméthylaminométhyl)-3-hydroxy-3-phényl-pentaméthylène)-7-fluoro-3,4-dihydro-1*H*-2,9-diazafluorène
sous forme du racémate ; des énantiomères, des diastéréoisomères, de mélanges des énantiomères ou des diastéréoisomères ou sous forme d'un énantiomère ou d'un diastéréoisomère individuel ; des bases et/ou de sels d'acides ou de cations physiologiquement acceptables.

12. Procédé de production de dérivés spirocycliques de cyclohexane suivant la revendication 1, **caractérisé en ce qu'**un composé de départ de formule générale II 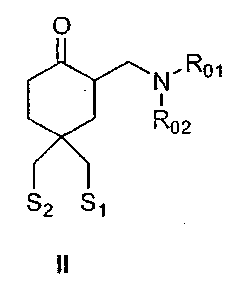 dans laquelle les restes R⁰¹ et R⁰² ont la définition indiquée pour R¹ et R² et peuvent en outre représenter un groupe protecteur, sont amenés à réagir dans un solvant organique avec un composé organométallique, avantageusement un composé de Grignard de formule générale R³MgX ou un composé organique de lithium R³-Li et après élimination du groupe protecteur cétal, le produit est amené à réagir avec un composé de formule générale B, dans laquelle X représente O ou S 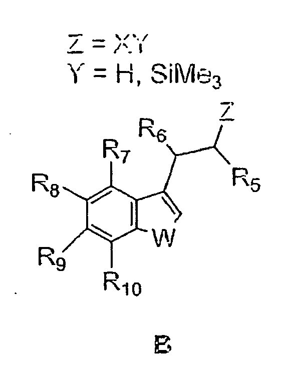 en présence de réactifs de couplage convenables, par exemple d'acides forts ou de leurs esters de silyle.

13. Procédé de production de dérivés spirocycliques de cyclohexane suivant la revendication 1, dans lesquels X représente un groupe NR¹⁷, **caractérisé en ce qu'**un composé de formule générale A 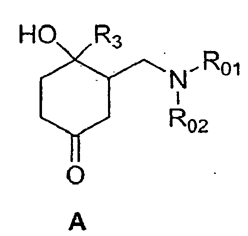 est amené à réagir avec un composé de formule générale Bₐ 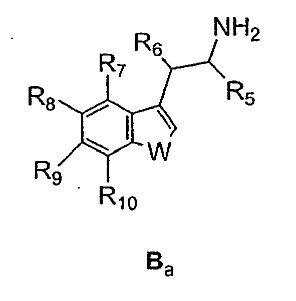 en présence de réactifs de couplage convenables, par exemple d'acides forts ou de leurs esters de silyle.

14. Procédé de production de dérivés spirocycliques de cyclohexane suivant la revendication 1, dans lesquels X représente un groupe NR¹⁷ et R¹⁷ est un reste COR¹² ou SO₂R¹², **caractérisé en ce qu'**un dérivé spirocyclique de cyclohexane dans lequel X représente NH, est amené à réagir avec un anhydride ou un chlorure d'acide, avec addition de base, par exemple de triéthylamine, avantageusement sous irradiation par micro-ondes.

15. Procédé de production de dérivés spirocycliques de cyclohexane suivant la revendication 1, dans lesquels X représente SO ou SO₂, **caractérisé en ce qu'**un dérivé spirocyclique de cyclohexane dans lequel X représente S, est oxydé à l'aide d'un agent oxydant, par exemple H₂SO₂.

16. Médicament contenant au moins un dérivé spirocyclique de cyclohexane suivant la revendication 1, éventuellement sous forme de son racémate, des stéréoisomères purs, en particulier des énantiomères et des diastéréoisomères, dans un rapport de mélange quelconque, sous forme de ses acides ou de ses bases ou sous forme de ses sels, en particulier des sels physiologiquement acceptables ou de sels d'acides ou de cations physiologiquement acceptables ; ou sous forme de ses produits de solvatation, en particulier des hydrates, contenant de même le cas échéant des additifs et/ou des substances auxiliaires convenables et/ou éventuellement d'autres substances actives.

17. Utilisation d'un dérivé spirocyclique de cyclohexane suivant la revendication 1, éventuellement sous forme de ses racémates, des stéréoisomères purs, en particulier des énantiomères et des diastéréoisomères, dans un rapport de mélange quelconque ; sous forme de ses acides
ou de ses bases ou sous forme de ses sels, en particulier des sels physiologiquement acceptables ou de sels d'acides
ou de cations physiologiquement acceptables ; ou sous forme de ses produits de solvatation, en particulier des hydrates ; pour la préparation d'un médicament destiné au traitement de la douleur, en particulier d'une douleur aiguë, neuropathique ou chronique.

18. Utilisation d'un dérivé spirocyclique de cyclohexane suivant la revendication 1, pour la préparation d'un médicament destiné au traitement d'angoisses, de dépressions, de l'épilepsie, de maladies cardiovasculaires, de l'incontinence urinaire, de la diarrhée, du prurit, de l'abus d'alcool et de drogues, de la pharmacodépendance, d'inflammations, de l'absence de motivation, de la boulimie, de l'anorexie, de la catalepsie, destiné à être utilisé comme anesthésique local, anti-arythmique, antiémétique, nootrope, et destiné à accroître la vigilance et la libido.
